# EUROPÄISCHE PATENTSCHRIFT

(11) **EP 4 384 019 B1**
(45) Veröffentlichungstag und Bekanntmachung des Hinweises auf die Patenterteilung: **25.06.2025**
(21) Anmeldenummer: 22765087.6
(22) Anmeldetag: 12.08.2022
(51) Int. Cl.: A23F 5/16, A23L 5/20, A23L 7/104, C12N 9/80

(54) **VERFAHREN ZUM ENTFERNEN VON ACRYLAMID AUS LEBENS- UND GENUSSMITTELN**
METHOD FOR THE REMOVAL OF ACRYLAMIDE FROM FOODS AND STIMULANTS
PROCÉDÉ D'ÉLIMINATION D'ACRYLAMIDE À PARTIR D'ALIMENTS ET DE STIMULANTS

(30) Priorität: 12.08.2021 WO PCT/EP2021/072477
(43) Veröffentlichungstag der Anmeldung: 19.06.2024
(73) Patentinhaber: ANKA Angewandte Kaffeetechnologie GmbH, 28237 Bremen (DE); c-LEcta GmbH, 04103 Leipzig (DE)
(72) Erfinder: PANTELIC, Nikolina, 28215 Bremen (DE); HOLTMANN, Meike, 28359 Bremen (DE); SÜSSE-HERRMANN, Oliver, 27798 Hude (DE); HOFFMANN, Gregor, 04416 Markkleeberg (DE); SCHÖNERT, Stefan, 04277 Leipzig (DE)
(74) Vertreter: Eisenführ Speiser
(86) Internationale Anmeldenummer: PCT/EP2022/072660
(87) Internationale Veröffentlichungsnummer: WO 2023/017147

(56) Entgegenhaltungen:
- WO-A1-2013/005145
- WO-A1-2021/123163
- WO-A1-2021/148508

## Beschreibung

### Technisches Gebiet

Die vorliegende Erfindung befindet sich auf dem Gebiet der Herstellung von Lebens- und Genussmitteln, insbesondere von Kaffee- und Kaffeeersatzprodukten mit verringertem Acrylamidgehalt. Es wird ein Verfahren zur Entfernung von Acrylamid aus verschiedenen Lebensmittelmatrices, insbesondere aus einer Kaffee- oder Kaffeeersatzproduktmatrix, bereitgestellt, welches die Verwendung eines Biokatalysators zum Abbau von Acrylamid beinhaltet.

### Hintergrund der Erfindung

Die Anforderungen von Endverbrauchern an ein konstant sicheres und unbedenklich verzehrbares Lebens- und Genussmittel sind kontinuierlich hoch und stellen spezielle Anforderungen an die Produzenten. Eine neue EU-Verordnung aus dem Jahr 2018 (EU 2017/2158) stuft Acrylamid als Prozesskontaminante und potentielles Gesundheitsrisiko für Endverbraucher ein. Demzufolge sind die Lebens- und Genussmittelproduzenten verpflichtet, den Acrylamidgehalt in Lebens- und Genussmitteln unter einem bestimmten Level zu halten oder zu senken. In Tierversuchen wirkt Acrylamid krebserzeugend und erbgutverändernd. Acrylamid entsteht in allen Brat-, Back- und Frittierprozessen von kohlenhydrathaltigen Rohstoffen und ist das Produkt der Erhitzung von Asparagin mit reduzierenden Zuckern (z.B. Glucose und Fructose) im Rahmen der sogenannten Maillard-Reaktion.

Im besonderen Fall der Produktion von Kaffee- und Kaffeeersatzprodukten beinhalten diese Prozesse das Brühen oder Extrahieren von gerösteten und gemahlenen Kaffeebohnen und deren Ersatzprodukten, wie z.B. Zichorie, Gerste oder Roggen. Beim Rösten werden die Kaffeebohnen typischerweise Temperaturen zwischen 145°C und 250°C unterzogen, wobei komplexe chemische Reaktionen, die Maillard-Reaktion, Karamellisierung und Pyrolyse erfolgen. Durch diese Reaktionen verändern sich die chemischen, physischen und sensorischen Eigenschaften der gerösteten Produkte, welche elementar wichtig für den Geschmack des Endproduktes sind. Zudem entstehen weitere Substanzen oder werden freigesetzt, die für das Endprodukt wichtig sind, wie zum Beispiel Antioxidantien (Jin et al. "Relationship between antioxidants and acrylamide formation: A review", Food Research International, 2013, p. 611 - 620). Als unerwünschte Prozesskontaminante durch das Rösten von Kaffee und Kaffeeersatzprodukten wird auch hier Acrylamid gebildet (Anese, M. "Acrylamid in Coffee and Coffee Substitutes, Acrylamid in Food, 2016, p.181 - 195). Die Richtwerte für Acrylamid der neuen EU-Verordnung liegen für Röstkaffee bei 400 µg/kg, für löslichen Kaffee bei 850 µg/kg, für Kaffeeersatzprodukte ausschließlich aus Getreide bei 500 µg/kg und für Produkte aus Zichorie bei 4000 µg/kg.

Acrylamid entsteht auch bei einer Vielzahl von (anderen) Prozessen in der Lebens- und Genussmittelindustrie. So entsteht beispielsweise beim Frittieren von Kartoffeln Acrylamid. Es ist vorteilhaft oder ggf. sogar nötig, dieses aus der Halbfertigware oder Fertigware teilweise oder vollständig zu entfernen, insbesondere um einerseits den Anforderungen der EU Verordnung (EU 2017/2158) zu genügen und andererseits ein sicheres und für den Verbraucher unbedenkliches Endprodukt zu erhalten.

Es existiert eine Vielzahl von Verfahren, welche das Entfernen von Acrylamid aus einer Zubereitung mittels Enzymen beschreiben. So offenbart die internationale Anmeldung WO 2004/083423 A1 ein Verfahren zum Abbau von Acrylamid in einer Zubereitung durch Verwendung einer Amidase. Auch die europäische Anmeldung EP 0272025 A2 betrifft ein Verfahren zur Zersetzung von Acrylamid unter Verwendung einer Amidase.

Weiterhin offenbart die internationale Patentanmeldung WO 2021/148508 ein Verfahren zum Abbau von Acrylamid in einer Zubereitung unter Verwendung einer besonders thermo- und pH-stabilen Amidase. Die Anmeldung offenbart keine Aufteilung der wässrigen Zubereitungen durch Membranverfahren, um nur einen Teilstrom mit einem Enzym zu behandeln.

Alle beschriebenen Verfahren sind solche, bei denen das verwendete Enzym in der Zubereitung zurückbleibt.

Um ein sicheres Lebens- oder Genussmittel für den Verbraucher zu erzielen, werden die verwendeten Enzyme meistens am Ende des Herstellungsprozesses durch Hitze inaktiviert und liegen nicht mehr in ihrer aktiven Form vor. Das verwendete Enzym, auch Biokatalysator genannt, kann daher nicht wiederverwendet werden. Zwar lassen sich heutzutage Enzyme kostengünstig herstellen, jedoch werden vor allem in der großmaßstäblichen Produktion von Lebens- und Genussmitteln große Mengen Enzym benötigt, sodass eine Wiederverwendung des eingesetzten Enzyms neben Kostengründen auch aus Nachhaltigkeitsgründen wünschenswert ist.

So beschreibt die internationale Anmeldung WO 2016/004949 A1 ein Verfahren zur Herstellung eines Kaffeeproduktes umfassend eine Enzymbehandlung von Kaffeeextrakt, bei der das verwendete Enzym in der Zubereitung thermisch inaktiviert wird oder über eine Membran zurückgehalten wird. Die Enzymbehandlung und anschließende Nachbehandlung findet im Kaffeeextrakt statt. In diesem liegen z.B. neben Aromastoffen auch partikuläre Komponenten vor, die (ebenfalls) wichtig für die sensorischen Eigenschaften des finalen Kaffeeprodukts sind. Bei einer Abtrennung des verwendeten Enzyms über eine Membran, werden diese wichtigen Komponenten abgetrennt und sind nicht mehr im Endprodukt vorhanden. Auch die internationalen Anmeldungen WO 2007/011531 A1 und WO 2016/207384 A1 offenbaren die Zurückhaltung eines Enzyms, sodass dieses nicht inaktiviert wird und wiederverwendet werden kann. Die Enzymbehandlung erfolgt hier im Kaffeeextrakt und die partikulären Bestandteile werden bei einer eventuellen Membranabtrennung mit abgetrennt. Weiterhin offenbaren alle vorstehend genannten Anmeldungen Kohlenhydrat-abbauende Enzyme zur Herstellung von Kaffeezubereitungen. Keine dieser Anmeldungen offenbart ein Verfahren zur Entfernung von Acrylamid.

Die internationale Anmeldung WO 2013/005145 beschreibt die enzymatische Entfernung der Acrylamidvorläufer Asparagin und Aspartat vor dem Rösten der Bohnen. Der beschriebene Prozess umfasst eine Extraktion der Bohnen mit Wasser, die enzymatischen Behandlung des wässrigen Extrakts mittels Asparaginase und Aspartase, die Konzentrierung des Extrakts, die Mischung des konzentrierten Extrakts mit den behandelten grünen Bohnen und die anschließende Trocknung. Erst anschließend erfolgt die Röstung und Weiterverarbeitung des Extrakts, wobei während des Röstvorgangs weniger Acrylamid gebildet wird, da die Reaktanden fehlen. Das Verfahren zeichnet sich durch zusätzlichen Wasser- und Energieverbrauch sowie den zusätzlichen apparativen Aufwand im vorgelagerten Schritt der wässrigen Extraktion und enzymatischen Behandlung aus und ist insgesamt wenig nachhaltig. Bei diesem Verfahren besteht die Gefahr, dass extrahierte Komponenten, deren Rückführung für die Herstellung eines geschmacklich und sensorisch einwandfreien Kaffees notwendig sind, durch die Konzentrierung oder Trocknung verloren gehen, da Komponenten über den notwendigen Abwasser- und Abluftstrom ausgetragen werden können, und somit die organoleptischen Eigenschaften des Endproduktes verändert werden.

Im Gegensatz dazu kann das erfindungsgemäße Verfahren leicht in die bestehenden Prozesse der Löskaffeeherstellung integriert werden, und umfasst keinen Auslass bzw. keine Abfallströme, über den ein Austragen von sensorisch oder geschmacklich wichtigen Komponenten erfolgen könnte.

Die Anmeldung WO 2021/123163 beschreibt ein Verfahren zur Herstellung eines flüssigen Kaffeekonzentrats mit reduziertem Acrylamidgehalt, wobei eine Abtrennung von Acrylamid aus einem ersten schwach aromatischen wässrigen Kaffeeextraktes durch eine selektivpermeable Membran erfolgt, wodurch ein zweiter schwach aromatischer wässriger Kaffeeextrakt mit einem niedrigeren Acrylamidgehalt als dem ersten schwach aromatischen wässrigen Kaffeeextraktes entsteht, und der zweite schwach aromatische wässrige Kaffeeextrakt in der Folge mit einem stark aromatischen wässrigen Kaffeeextrakt kombiniert wird. Eine enzymatische Behandlung eines der wässrigen Kaffeeextrakte ist nicht vorgesehen.

Bei einer enzymatischen Behandlung von Zubereitungen, die vor allem aufgrund ihres Geschmacks oder Geruchs verzehrt oder verwendet werden, ist es wichtig, alle Stoffe und Komponenten während der Enzymbehandlung zu bewahren, die zum Aroma, zum Geschmack oder zum Geruch beitragen, um ein geschmacklich und olfaktorisch einwandfreies Endprodukt zu erzielen. Prozesse zur enzymatischen Behandlung von Zubereitungen sowie Hydrolysierungsreaktionen wie in den obenstehend beschriebenen Anmeldungen offenbart, besitzen unter Umständen den Nachteil, dass bei Verwendung solcher Enzyme auch Nebenreaktionen oder Überreaktionen auftreten, die zum Beispiel die gewünschten Komponenten solcher Zubereitungen umwandeln oder abbauen. Weiterhin besteht bei den aus dem Stand der Technik bekannten Verfahren die Gefahr der unerwünschten Abtrennung von weiteren, für die sensorischen Eigenschaften des Endproduktes wichtigen weiteren Komponenten, insbesondere, wenn die zur Behandlung eingesetzten Enzyme inaktiviert oder entfernt werden. Hierbei entsteht dann ein Endprodukt, welches in seinen Eigenschaften von dem gewünschten Endprodukt abweicht.

Daher war es die primäre Aufgabe der vorliegenden Erfindung, ein Verfahren bereitzustellen, welches ein sicheres Endprodukt mit reduziertem Gehalt an Acrylamid bereitstellt und gleichzeitig die für die gewünschten geschmacklichen und olfaktorischen Eigenschaften des gewünschten Endprodukts notwendigen Stoffe und Komponenten bewahrt, vorzugsweise ohne dass dabei die zur Reduktion an Acrylamid eingesetzten Enzyme im Endprodukt verbleiben. Weiterhin soll das Verfahren ökonomisch im Großmaßstab angewandt werden.

### Beschreibung der Erfindung

Diese primäre Aufgabe wird gemäß der vorliegenden Erfindung gelöst durch das Bereitstellen eines Verfahrens zum Entfernen oder Umsetzen von Acrylamid aus einer wässrigen Zubereitung zur Herstellung eines Endprodukts mit verringertem Gehalt an Acrylamid, umfassend die folgenden Schritte:
(i) Bereitstellen einer wässrigen Zubereitung enthaltend Acrylamid und weitere Komponenten als Ausgangsprodukt zur Herstellung eines Endprodukts mit verringertem Gehalt an Acrylamid;
(ii) Aufteilen der wässrigen Zubereitung in zwei Stoffströme, um ein Retentat (R1) und ein Permeat (P1) zu erhalten, wobei das Retentat (R1) die weiteren Komponenten (K1) enthält, und wobei das Permeat (P1) Acrylamid und vorzugsweise keine oder einen geringen Anteil an weiteren Komponenten (K2) enthält;
(iii) in Kontakt bringen des Permeats (P1) mit einem Acrylamid reduzierenden Biokatalysator und Erhalten eines Permeats (P2) mit reduziertem Gehalt an Acrylamid;
(iv) Zusammenführen des erhaltenen Permeats (P2) mit reduziertem Gehalt an Acrylamid und des Retentats (R1) aus Schritt (ii) und Erhalten einer wässrigen Zubereitung mit reduziertem Gehalt an Acrylamid, wobei das Permeat (P2) keinen Biokatalysator enthält;
(v) optional, Weiterverarbeiten der wässrigen Zubereitung mit reduziertem Gehalt an Acrylamid aus Schritt (iv), um ein Endprodukt zu erhalten.

Eine wässrige Zubereitung im Kontext der vorliegenden Erfindung ist vorzugsweise eine Zubereitung, die einen Wassergehalt von ≥ 50 Gew.-%, vorzugsweise ≥ 70 Gew.-% und besonders bevorzugt mehr als ≥ 90 Gew.-%, bezogen auf das Gesamtgewicht der Zubereitung, besitzt.

Bevorzugt einzusetzende wässrige Zubereitungen im Kontext der vorliegenden Erfindung sind Kaffee- oder Kaffeeersatzextrakte in unkonzentrierter oder konzentrierter Form, Fruchtsäfte oder Fruchtzubereitungen, Limonaden, Teeextrakte, Milch- oder Milchproduktzubereitungen und breiige Massen, vorzugsweise zur Herstellung von Frühstückscerealien.

"Kaffeeextrakte" im Kontext der vorliegenden Erfindung sind Extrakte von gerösteten und gemahlenen Kaffeebohnen.

"Kaffeeersatzextrakte" im Kontext der vorliegenden Erfindung sind Extrakte von gerösteten und gemahlenen Ersatzprodukten von Kaffeebohnen, wie z.B. Zichorie, Gerste, Lupine, Ginseng, Reishi, Dinkel, Mais, Löwenzahnwurzel oder Roggen.

Das in der wässrigen Zubereitung enthaltene Acrylamid kann das Verfahrensprodukt der Maillard-Reaktion sein, wie oben beschrieben. Acrylamid (Prop-2-enamid) besitzt ein Molekulargewicht von 71,08 g/mol beziehungsweise 71,08 Da.

Weiterhin sind in der wässrigen Zubereitung Komponenten enthalten, welche eine Funktion als Geschmacks- oder Geruchsgeber der Zubereitung erfüllen. Weiterhin können bestimmte enthaltene Komponente der wässrigen Zubereitung zu einem angenehmen, erwünschten Mundgefühl der Zubereitung beitragen.

Der Begriff Komponenten im Kontext der vorliegenden Erfindung bezeichnet weitere Stoffe außer Acrylamid, welche entweder gelöst oder als Partikel vorliegen können. Partikel im Sinne im Sinne der Erfindung sind sowohl suspendierte Partikel (Partikel im eigentlichen Sinne) als auch kolloidal oder dispers gelöste Partikel. Partikel können auch weitere gelöste Stoffe wie zum Beispiel Aromastoffe, Polysaccharide wie Lignin, Proteine oder Fette sein.

Komponenten im Sinne der Erfindung bezeichnet alle Partikel und gelösten Stoffe sofern sie der jeweils entsprechenden erfindungsgemäßen Partikel- oder Komponentengröße entsprechend. Die vorliegenden Partikel können eine mittlere Partikelgröße von unter 50 mm, vorzugsweise unter 5 mm, besonders bevorzugt im Bereich von 0,3 - 100 µm, besitzen. Vorzugsweise können die vorliegenden Partikel eine Partikelgröße im Bereich von 1 - 100 µm besitzen. Sie sind für das Mundgefühl des Produktes von elementarer Bedeutung. Insbesondere in Kaffee- und Kaffeeersatzzubereitungen geben diese Stoffe dem Endprodukt seinen charakteristischen Geruch und Geschmack und sollen daher erhalten bleiben.

Die in einer eingesetzten wässrigen Zubereitung befindlichen Partikel, besonders Polysaccharide und Proteine, können durch enzymatische oder thermische Hydrolysierungsreaktionen abgebaut werden. Die vorliegende Erfindung stellt ein Verfahren bereit, mittels welchem Komponenten, also Partikel und gelöste Stoffe, mit einer gewissen Größe abgetrennt und vor ungewünschten enzymatischen oder thermischen Hydrolysierungsreaktionen geschützt werden können.

Die in der eingesetzten wässrigen Zubereitung vorhandenen Komponenten werden als (K1) und (K2) bezeichnet. Die Komponenten K1 sind Partikel und gelöste Stoffe mit einer Größe über 0,01 µm, bevorzugt über 0,1 µm, bevorzugt über 1 µm, weiterhin bevorzugt über 10 µm, vorzugsweise über 100 µm und weiterhin bevorzugt über 500 µm. Die Komponenten (K2) sind alle Partikel und gelösten Stoffe mit einer Größe kleiner 0,01 µm, bevorzugt kleiner 0,1 µm, weiterhin bevorzugt kleiner 1 µm, vorzugsweise kleiner 10 µm und weiterhin bevorzugt kleiner 100 µm. Die eingesetzte wässrige Zubereitung kann in zwei Stoffströme getrennt werden, die entweder (K1) oder (K2) enthalten. Das Zusammenführen der Stoffströme im Schritt (iv), welche die Komponenten (K1) und (K2) enthalten, resultiert in einer Mischung, welche mit Ausnahme des Acrylamids und der Umsetzungsprodukte des Acrylamids der ursprünglich eingesetzten wässrigen Zubereitung entspricht.

Die Begriffe Permeat und Retentat im Kontext der vorliegenden Erfindung beschreiben die erhaltenen Stoffströme nach Trennung einer Ausgangsmischung über einen physikalischen Vorgang. Dabei ist das Retentat der Teil der Ausgangsmischung, der bei diesem Vorgang zurückgehalten wird und das Permeat der Teil der Ausgangsmischung, welcher nicht zurückgehalten wird.

Ein bevorzugter physikalischer Vorgang zur Erzeugung eines Permeats und eines Retentats ist die Trennung eines Stoffstroms mittels einer Membran. In einer bevorzugten Ausführungsform kann ein aus einer Ausgangsmischung mittels einer ersten physikalischen Trennung erhaltenes Permeat durch eine zweite physikalische Trennung weiterhin in ein weiteres Retentat und ein weiteres Permeat aufgeteilt werden. In einer bevorzugten Ausführungsform unterscheiden sich dabei die Trennungsparameter der ersten physikalischen Trennung von den Trennungsparametern der zweiten physikalischen Trennung. In einer alternativen bevorzugten Ausführungsform unterscheiden sich dabei die Trennungsparameter der ersten physikalischen Trennung und der zweiten physikalischen Trennung nicht.

Die Zusammensetzung von (K1) und (K2) wird dabei durch die Art und Porengröße einer ersten Membran (M1) bestimmt. Der Stoffstrom des Permeats wird dabei über einen Druckgradienten, oder einen Konzentrationsgradienten, oder durch einen kombinierten Druck- und Konzentrationsgradienten angetrieben.

Die Partikel und gelösten Stoffe der Komponenten (K1) bleiben im Retentat (R1) zurück und befinden sich nicht im Permeat (P1). Vorzugsweise werden die verbleibenden Komponenten (K1) nicht in Kontakt mit dem Biokatalysator gebracht. Weiterhin bevorzugt ist es im Kontext der vorliegenden Erfindung, dass die Komponenten (K1) partikulär oder gelöste Stoffe sind und/oder solche sind, die bestimmte sensorische Eigenschaften im Endprodukt erfüllen, wie z.B. ein angenehmes Mundgefühl vermitteln bzw. dazu beitragen. Die Komponenten (K2) besitzen eine kleinere Größe, vorzugsweise eine vergleichbare Größe zu Acrylamid, und befinden sich nach Trennung der Stoffströme in Schritt (ii) im Permeat (P1). Es ist bevorzugt im Rahmen der vorliegenden Erfindung, dass die Komponenten (K2) nach dem in Kontakt bringen des Permeats (P1) mit dem Biokatalysator und dessen Überführung in das Permeat (P2) in Schritt (iii) mit dem Schritt (iv) in die wässrige Zubereitung rückgeführt werden. Weiterhin ist es bevorzugt, dass die Komponenten (K2) kleiner als der Biokatalysator sind.

"Aufteilen der Stoffströme" im Kontext der vorliegenden Erfindung beschreibt das Aufteilen der wässrigen Zubereitung in einen verbleibenden Teil, das sogenannte Retentat (R1), in welchem sich die zusätzlichen, verbleibenden Komponenten (K1) befinden und in einen Teil Permeat (P1), in welchem sich neben Wasser Acrylamid sowie gegebenenfalls ein geringer Anteil an gelösten weiteren Komponenten (K2) mit einer ähnlichen Größe beziehungsweise mit einem ähnlichen Molekulargewicht wie Acrylamid befindet. Vorzugsweise ist das Permeat (P1) frei oder im Wesentlichen frei von weiteren Komponenten, die ungelöste Partikel darstellen. Die Zusammensetzung der Komponenten (K2) im Permeat (P1) ergibt sich aus der Art und Porengröße der verwendeten Membran (M1).

In einer bevorzugten Ausführungsform sind die Art und Porengröße der Membran (M1) so gewählt, dass Acrylamid die Membran (M1) passieren kann. In einer weiteren bevorzugten Ausführungsform sind die Art und Porengröße der Membran (M1) so gewählt, dass der Biokatalysator die Membran (M1) nicht passieren kann.

"In Kontakt bringen" im Rahmen der vorliegenden Erfindung bedeutet, dass das im Permeat (P1) enthaltene Acrylamid in Kontakt mit dem Biokatalysator kommt und so von diesem umgesetzt werden kann. Vorzugsweise geschieht die Umsetzung im Rahmen der vorliegenden Erfindung durch Hydrolyse, sodass unschädliche Acrylsäure gebildet wird. Der erhaltene Stoffstrom nach Enzymbehandlung (Permeat (P2)) besitzt einen reduzierten Gehalt an Acrylamid. Entsprechend bezeichnet ein "Acrylamid reduzierender Biokatalysator" im Rahmen der vorliegenden Erfindung einen Biokatalysator, der Acrylamid enzymatisch in ein anderes Produkt umwandelt oder umsetzt und damit den Gehalt oder die Konzentration des Acrylamids verringert bzw. reduziert.

Vorzugsweise wird im Rahmen der vorliegenden Erfindung das Permeat (P2) erhalten, indem der Stoffstrom nach dem in Kontakt bringen des Permeats (P1) mit dem Biokatalysator in Schritt (iii) und vor dem Zusammenführen von Permeat (P2) und dem Retentat R1 in Schritt (iv) von dem Biokatalysator abgetrennt wird. Die Abtrennung des Biokatalysators erfolgt vorzugsweise mittels Separation, z.B. Zentrifugation, Sedimentation, oder Filtration über ein Sieb, über ein Gewebe oder über eine Membran. Besonders bevorzugt im Kontext der vorliegenden Erfindung erfolgt die Abtrennung des Biokatalysators über eine Membran (M2).

Das bei der Abtrennung des Biokatalysators erhaltene Retentat (R2) beinhaltet den Biokatalysator und das Permeat (P2) beinhaltet keinen Biokatalysator.

In einer bevorzugten Ausführungsform der vorliegenden Erfindung umfassen die Schritte (iii) und (iv) des erfindungsgemäßen Verfahrens die folgenden Teilschritte:
(iii-1) in Kontakt bringen des Permeats (P1) mit einem Acrylamid reduzierenden Biokatalysator und Erhalten eines Permeats (P2) mit reduziertem Gehalt an Acrylamid wobei das Permeat (P2) den Biokatalysator umfasst;
(iii-2) Abtrennung des Biokatalysators aus dem Permeat (P2) aus Schritt (iii-1) mittels Separation über eine Membran (M2) und Erhalt eines Retentats (R2), das den Biokatalysator beinhaltet und Erhalt des Permeats (P2) mit reduziertem Gehalt an Acrylamid wobei das Permeat (P2) keinen Biokatalysator umfasst;
(iv) Zusammenführen des erhaltenen Permeats (P2) mit reduziertem Gehalt an Acrylamid aus Schritt (iii-2) und des Retentats (R1) aus Schritt (ii) und Erhalten einer wässrigen Zubereitung mit reduziertem Gehalt an Acrylamid.

In einer bevorzugten Ausführungsform der vorliegenden Erfindung erfolgen die Schritte (ii) und (iii-2) des erfindungsgemäßen Verfahrens über zwei verschiedene Membranen (M1) und (M2), wobei sich Art und Porengröße der Membranen (M1) und (M2) unterscheiden kann und derart gewählt ist, dass der Biokatalysator die Membran (M1) nicht passieren kann.

In einer bevorzugten Ausführungsform der vorliegenden Erfindung erfolgen die Schritte (ii) und (iii-2) des erfindungsgemäßen Verfahrens über eine einzige Membran (M1) wobei sich Art und Porengröße der Membran (M1) derart gewählt ist, dass der Biokatalysator die Membran (M1) nicht passieren kann.

In einer bevorzugten Ausführungsform der Erfindung ist die Porengröße der Membran (M2), welche den Biokatalysator zurückhalten soll, kleiner als die Größe des Biokatalysators. In einer besonders bevorzugten Ausführungsform ist die Porengröße der Membran (M2) um den Faktor 1,5 kleiner als der Biokatalysator. Weiterhin ist es zu bevorzugen, dass die Porengröße der Membran um den Faktor 2 kleiner ist als der Biokatalysator, noch bevorzugter ist es, wenn die Porengröße der Membran um den Faktor 2,5 kleiner ist als der Biokatalysator und am bevorzugtesten ist es, wenn die Porengröße der Membran um den Faktor 3 oder mehr kleiner ist als der Biokatalysator.

In einer bevorzugten Ausführungsform erfolgt die Abtrennung des Biokatalysators aus dem Permeat (P2) wie oben beschrieben über eine Membran (M2), welche eine Porengröße besitzt, die größer ist als die Größe der im Permeat (P2) enthaltenen Komponenten (K2), und kleiner ist als die im Retentat (R1) enthaltenen Komponenten (K1), und kleiner ist als der Biokatalysator.

In einer weiteren bevorzugten Ausführungsform sind die Porengrößen der Membran (M1) kleiner oder gleich den Poren der Membran (M2).

In einer weiteren bevorzugten Ausführungsform sind die Porengrößen der Membran (M1) kleiner oder gleich den Poren der Membran (M2), und die Porengrößen von Membran (M1) und Membran (M2) sind jeweils kleiner als der Biokatalysator.

Besonders bevorzugt im Rahmen der vorliegenden Erfindung ist es, wenn die Abtrennung des Biokatalysators über eine Membran mit einer Porengröße von d₉₀ < 100 kDa, bevorzugt d₉₀ < 50 kDa, besonders bevorzugt d₉₀ ≤ 30 kDa, durchgeführt wird, besonders bevorzugt d₉₀ ≤ 10 kDa.

In Schritt (iv) des Verfahrens gemäß der vorliegenden Erfindung wird das erhaltene Permeat (P2) mit reduziertem Gehalt an Acrylamid und enthaltend die Komponenten (K2) zu dem Retentat (R1) enthaltend die weiteren Komponenten (K1) hinzugefügt, sodass eine wässrige Zubereitung mit einem verringerten Gehalt an Acrylamid im Vergleich zum Gehalt an Acrylamid der eingesetzten wässrigen Zubereitung aus Schritt (i) erhalten wird.

Vorzugsweise werden die Schritte des Verfahrens iterativ, besonders bevorzugt kontinuierlich, durchgeführt, bis die gewünschte Reduktion an Acrylamid erreicht wurde.

In einer bevorzugten Ausführungsform erfolgen die Schritte (i), (ii), (iii-1), (iii-2), und (iv) des erfindungsgemäßen Verfahrens iterativ, besonders bevorzugt kontinuierlich, über zwei Membranen (M1) und (M2) bis die gewünschte Reduktion an Acrylamid erreicht wurde.

In einer weiteren bevorzugten Ausführungsform erfolgen die Schritte (i), (ii), (iii-1), (iii-2), und (iv) des erfindungsgemäßen Verfahrens iterativ, besonders bevorzugt kontinuierlich, über eine Membran (M1) bis die gewünschte Reduktion an Acrylamid erreicht wurde.

In einer weiteren bevorzugten Ausführungsform erfolgen Schritt (ii), (iii), und (iv) des erfindungsgemäßen Verfahrens über eine einzige Membran (M1), umfassen die folgenden Schritte:
(ii) Aufteilen der wässrigen Zubereitung in zwei Stoffströme mittels einer Membran (M1), um ein Retentat (R1) und ein Permeat (P1) zu erhalten, wobei das Retentat (R1) die weiteren Komponenten (K1) enthält, und wobei das Permeat (P1) Acrylamid und vorzugsweise keine oder einen geringen Anteil an weiteren Komponenten (K2) enthält;
(iii-1) in Kontakt bringen des Permeats (P1) mit einem Acrylamid reduzierenden Biokatalysator und Erhalten eines Permeats (P2) mit reduziertem Gehalt an Acrylamid wobei das Permeat (P2) den Biokatalysator umfasst;
(iii-2) Abtrennung des Biokatalysators aus dem Permeat (P2) aus Schritt (iii-1) mittels Separation über eine Membran (M1) und Erhalt eines Retentats (R2), das den Biokatalysator beinhaltet und Erhalt des Permeats (P2) mit reduziertem Gehalt an Acrylamid wobei das Permeat (P2) keinen Biokatalysator umfasst;
(iv) Zusammenführen des erhaltenen Permeats (P2) mit reduziertem Gehalt an Acrylamid aus Schritt (iii-2) und des Retentats (R1) aus Schritt (ii) und Erhalten einer wässrigen Zubereitung mit reduziertem Gehalt an Acrylamid;
wobei die Schritte (iii-2) und (iv) gleichzeitig erfolgen.

In einer bevorzugten Ausführungsform erfolgen die Schritte (ii), (iii-1), (iii-2), und (iv) kontinuierlich über die Membran (M1). Vorzugsweise erfolgt die Aufteilung in zwei Stoffströme in Schritt (ii) sowie die Zusammenführung von Permeat P2 und Retentat (R1) mittels Diffusion von Acrylamid entsprechend dessen Diffusionsgradienten über die Membran (M1).

In einer bevorzugten Ausführungsform des erfindungsgemäßen Verfahrens wird das in Kontakt bringen in Schritt (iii) bei Umgebungstemperatur, vorzugsweise bei einer Temperatur von über 30 °C, bevorzugt von über 40°C, weiterhin bevorzugt von über 50 °C, vorzugsweise bei einer Temperatur von über 60 °C, weiterhin bevorzugt bei einer Temperatur von über 70°C und auch weiterhin bevorzugt bei einer Temperatur von über 80°C durchgeführt.

Die hohen Temperaturen, welche für die Enzymaktivität und den Acrylamidabbau erforderlich sind, können sich bei einer Zeit über 30 Minuten negativ auf die Komponenten (K1) der wässrigen Zubereitung auswirken. Daher ist es besonders bevorzugt, diese in einem ersten Schritt abzutrennen, sodass diese im Retentat (R1) verbleiben und nicht mit erhöhten Temperaturen sowie dem Biokatalysator in Berührung kommen.

In einer weiteren bevorzugten Ausführungsform des erfindungsgemäßen Verfahrens wird das in Kontakt bringen in Schritt (iii) bei einem pH-Wert in einem Bereich von pH 4 bis pH 7, vorzugsweise in einem Bereich von pH 4 bis pH 6,5, vorzugsweise im Bereich von pH 4,5 bis pH 5,5 durchgeführt.

In einer bevorzugten Ausführungsform des erfindungsgemäßen Verfahrens wird das in Kontakt bringen in Schritt (iii) für eine Zeit von mehr als 10 Minuten, vorzugsweise von mehr als 15 Minuten, weiterhin bevorzugt von mehr als 30 Minuten, bevorzugt von mehr als einer Stunde, weiterhin bevorzugt von mehr als 2 Stunden, bevorzugt von mehr als 4 Stunden, weiterhin bevorzugt von mehr als 8 Stunden, bevorzugt von mehr als 12 Stunden und besonders bevorzugt von mehr als 16 Stunden durchgeführt.

Mit dem vorliegenden Verfahren kann ein Endprodukt hergestellt werden, welches einen verringerten Gehalt an Acrylamid besitzt, welcher die Erfordernisse an ein sicheres Lebens- und Genussmittel erfüllt und vorzugsweise die Bestimmungen der EU-Verordnung 2017/2158 erfüllt. Zusätzlich besitzt das erhaltene Endprodukt vorteilhafterweise das gleiche oder im Wesentlichen das gleiche Geschmacks- und Geruchsprofil wie eine nicht behandelte (Kaffee-)Zubereitung.

In einer bevorzugten Ausführungsform der vorliegenden Erfindung betrifft diese ein Verfahren gemäß der vorliegenden Erfindung, wobei das Aufteilen der Stoffströme in Schritt (ii) als Filtration durchgeführt wird, vorzugweise als Membranfiltration.

Bei der Filtration im Kontext der vorliegenden Erfindung wird ein Ausgangsgemisch basierend auf der Größe der beinhalteten Partikel oder Komponenten aufgetrennt. Hierbei ist zu beachten, dass die Porengröße des Filtrationsmaterials so gewählt wird, dass die abzutrennenden Komponenten zurückbleiben. Eine Form der Filtration ist die Membranfiltration, bei der ein Stoffgemisch mittels einer Membran aufgetrennt wird. Bei einer Membranfiltration können bei geeigneter Wahl der Porengröße neben ungelöst vorliegenden Partikeln auch gelöst vorliegende Komponenten basierend auf ihrer Größe beziehungsweise ihres Molekulargewichts abgetrennt werden. Die Membranfiltration kann zum Beispiel als Gleichstrom- oder Gegenstromfiltration durchgeführt werden.

Vorzugsweise im Rahmen der vorliegenden Erfindung ist, wann immer auf eine Membran verwiesen wird, die Membrangeometrie ausgewählt aus der Gruppe bestehend aus Hohlfasermembran (hollow fibre membrane), Hohlfasermembranmodul (hollow fibre membrane module, Kapillarmembran (capillary membrane), Kapillarmembranmodul (capillary membrane module) Flachmembran (flat sheet membrane), Flachmembranmodul (flat sheet membrane module or plate und frame membrane module), Wickelmembranmodule, Spiralwickelmodul (spiral wound membrane module) oder Tubular- bzw. Rohrmodul (tubular membrane module or multichannel membrane module).

Weiterhin bevorzugt im Rahmen der vorliegenden Erfindung ist, wann immer auf eine Membran verwiesen wird, die Verwendung mindestens eines Membranmaterials ausgewählt aus der Gruppe bestehend aus Polysulfone, Polyethersulfon (PES) Cellulose, Celluloseester wie Celluloseacetat und Cellulosenitrat, regenerierte Cellulose (RC), Silikone, Polyamide, Polyamidimid, Polyamid Harnstoff, Polycarbonate, Keramik, Edelstahl, Silber, Silizium, Zeolith, Polyacrylnitril (PAN), Polyethylen (PE), Polypropylen (PP), Polytetrafluorethylen (PTFE), Polyvinylidenfluorid (PVDF), Polyvinylchlorid (PVC) und Polypiperazinamid.

Vorzugsweise können auch modifizierte Membranmaterialien wie vorhergehend definiert im Rahmen der vorliegenden Erfindung eingesetzt werden oder eine Kombination der vorstehend genannten Membranmaterialien und deren modifizierten Formen.

Eine weitere bevorzugte Ausführungsform des Verfahrens gemäß der vorliegenden Erfindung betrifft ein Verfahren, wobei das Aufteilen der Stoffströme in Schritt (ii) als Membranfiltration durchgeführt wird und die Porengröße der Membran d₉₀ ≤ 100 kDa, bevorzugt d₉₀ ≤ 50 kDa, besonders bevorzugt d₉₀ ≤ 30 kDa und besonders bevorzugt d₉₀ ≤ 10 kDa beträgt.

Eine weitere bevorzugte Ausführungsform des Verfahrens gemäß der vorliegenden Erfindung betrifft ein Verfahren, wobei das Aufteilen der Stoffströme in Schritt (ii) als Membranfiltration durchgeführt wird und die Membran eine Porengröße im Bereich von 0,01 bis 10 µm, bevorzugt im Bereich von 0,05 bis 5 µm und besonders bevorzugt im Bereich von 0,1 bis 1 µm, besitzt.

Vorzugsweise erfolgt das Aufteilen der Stoffströme in Schritt (ii) durch Einsatz einer Membran M1 mit einer Porengröße, die kleiner ist als die Größe der im Retentat (R1) enthaltenen verbleibenden Komponenten (K1), und kleiner ist als der Biokatalysator.

Vorzugsweise erfolgt die Abtrennung des Biokatalysators vor Schritt (iv) durch Einsatz einer Membran (M2) mit einer Porengröße, die kleiner ist als der Biokatalysator, und die größer oder gleich der im Permeat (P2) enthaltenen Komponenten (K2) ist. Vorzugsweise erfolgt das Aufteilen der Stoffströme in Schritt (ii) und das Zusammenführen der Prozessströme in Schritt (iv) durch Einsatz einer Membran M1 mit einer Porengröße, die kleiner ist als die Größe des Biokatalysators unter gleichzeitiger Abtrennung des Biokatalysators.

Die Porengröße einer Membran kann nach ihrem d₉₀-Wert bestimmt werden. Dieser sogenannte "Cut-Off" gibt die minimale Molekülmasse eines globulären Moleküls an, welches durch die Membran zu 90 % zurückgehalten wird.

Wiederum eine weitere bevorzugte Ausführungsform des Verfahrens gemäß der vorliegenden Erfindung betrifft ein Verfahren gemäß der vorliegenden Erfindung, wobei der Transmembrandruck zwischen 0,01 mbar und 10 bar, bevorzugt zwischen 0,1 mbar und 7,5 bar, weiterhin bevorzugt zwischen 1 mbar und 5 bar besonders bevorzugt zwischen 500 mbar und 2,5 bar liegt, und/oder gehalten wird.

Der Transmembrandruck spielt bei der Auslegung eines membrangetriebenen Prozesses eine Rolle. So unterscheiden sich grundsätzlich zwei Betriebsweisen: Arbeiten bei einem konstanten Transmembrandruck oder bei einer konstanten Flussrate. Im Rahmen der vorliegenden Erfindung ist es bevorzugt, wenn die Membranfiltration bei einer kontinuierlichen Flussrate betrieben wird, wobei sich der Transmembrandruck im oben genannten bevorzugten Bereich bewegt.

In einer weiteren Ausführungsform des vorliegenden Verfahrens ist es bevorzugt, wenn die Membranfiltration bei einem konstanten Transmembrandruck erfolgt und die Flussrate variabel ist.

In einer bevorzugten Ausführungsform des Verfahrens erfolgt das Verfahren unter Einsatz eines Transmembrandrucks (1) bei der Aufteilung der Stoffströme an Membran (M1) in Schritt (ii), oder bevorzugt, wenn die Abtrennung des Biokatalysators in Schritt (iii) über eine Membran erfolgt, unter Einsatz eines Transmembrandrucks (2) an Membran (M2) bei der Abtrennung des Biokatalysator in Schritt (iii), oder unter Einsatz eines Transmembrandrucks (3) bei Aufteilen der Stoffströme über eine Membran (M1) in Schritt (ii) und bei Abtrennen des Biokatalysators über eine Membran (M2) in Schritt (iii) des erfindungsgemäßen Verfahrens.

Eine bevorzugte Ausführungsform betrifft ein Verfahren gemäß der vorliegenden Erfindung, wobei das Endprodukt ein dem Genuss, der Ernährung dienendes oder kosmetisches Endprodukt ist, und bevorzugt ausgewählt ist aus der Gruppe umfassend gebratene oder frittierte Kartoffelprodukte, Maisprodukte, Kaffeeprodukte, Kaffeeersatzprodukte, Snacks, Weizenprodukte, Kosmetika, Feingebäck wie Plätzchen, Kekse, Zwieback, Getreideriegel, Scones, Eiswaffeln, Waffeln, Crumpets, Lebkuchen, Knäckebrot und Brotersatzprodukte, Nudeln, Reis, Fischerzeugnisse, Fleischerzeugnisse, Cerealien, Bier oder Beikost für Kinder und Säuglinge.

Ganz besonders bevorzugt im Rahmen der vorliegenden Erfindung ist das Endprodukt ein Kaffee- oder Kaffeeersatzprodukt.

Im Rahmen der vorliegenden Erfindung ist es weiterhin bevorzugt, wenn das Endprodukt ein der Tier-Ernährung dienendes Endprodukt ist, bevorzugt ausgewählt aus der Gruppe bestehend aus Futter, Nährstofflösungen, Nährstoffpräparate, Fruchtsäfte, Fruchtzubereitungen und Milchzubereitungen.

Wiederum eine bevorzugte Ausführungsform betrifft ein Verfahren gemäß der vorliegenden Erfindung, wobei der Biokatalysator als lebender Mikroorganismus, inaktivierter Mikroorganismus und/oder als Zelllysat und/oder als (teil)aufgereinigtes Enzym und/oder als immobilisiertes Enzym vorliegt.

In einer weiteren bevorzugten Ausführungsform liegt das Enzym als Dimer und/oder Multimer und/oder quervernetzt vor. Verfahren zur Dimerisierung und Multimerisierung von Enzymen sind dem Fachmann bekannt.

Das Enzym kann unabhängig von seiner Funktion unterschiedlich groß sein, z.B. durch unterschiedlich lange Polypeptidketten oder durch Bildung eines Zusammenschlusses von mehreren Enzymuntereinheiten zu einem aktiven Enzym. Enzyme, welche die Aktivität einer Amidase entsprechend der Erfindung vermitteln, können aus einzelnen Untereinheiten bestehen, die 15 bis 80 kDa groß sein können. Weiterhin gibt es Enzyme, die aus mehreren Untereinheiten bestehen und von 40 bis 400.000 kDa groß sein können (z.B. Uniprot P95896, Oktamer aus ca. 55 kDa UE). Enzyme gemäß der vorliegenden Erfindung können natürlicherweise als Dimer oder Multimer vorliegen oder mittels dem Fachmann bekannter Verfahren hergestellt werden.

Ein "lebender Mikroorganismus" im Kontext der vorliegenden Erfindung bezeichnet einen Mikroorganismus, welcher die Fähigkeit besitzt, sich zu teilen oder fortzupflanzen. Dieser Mikroorganismus exprimiert den Biokatalysator als Metabolit.

Ein "inaktivierter Mikroorganismus" im Kontext der vorliegenden Erfindung bezeichnet einen Mikroorganismus, welchem die Fähigkeit fehlt, sich zu teilen oder fortzupflanzen. Der Biokatalysator liegt im Inneren des Mikroorganismus oder als Membranprotein oder anderweitig mit der Membran des Mikroorganismus verbunden, vor und ist enzymatisch aktiv.

Ein "Zelllysat" im Kontext der vorliegenden Erfindung bezeichnet das Produkt eines Zellaufschlusses von einem Mikroorganismus, welcher den Biokatalysator exprimiert. Durch den Zellaufschluss verliert der Mikroorganismus seine Fähigkeit, sich fortzupflanzen oder zu teilen; die weiteren Zellbestandteile sind neben dem Biokatalysator im Zelllysat nachweisbar.

Ein "(teil)aufgereinigtes Enzym" im Kontext der vorliegenden Erfindung bezeichnet ein Enzym entstammend einem Zelllysat, in welchem keine oder nur wenige Zellbestandteile nachweisbar sind und der Gehalt des Enzyms über 1 Gew.-%, über 2 Gew.-%, über 5 Gew.-%, vorzugsweise über 10 Gew.-%, über 15 Gew.-%, über 20 Gew.-% und besonders bevorzugt über 30 Gew.-%, liegt.

In einer bevorzugten Ausführungsform liegt der Biokatalysator als Lyophilisat vor und wird als solcher in das Verfahren eingesetzt.

In einer bevorzugten Ausführungsform liegt der Biokatalysator als immobilisiertes Enzym vor. Geeignete Verfahren zur Enzymimmobilisierung umfassen Quervernetzung, Einschlussimmobilisierung, adsorptive oder kovalente Bindung an die Oberfläche eines Trägermaterials. Das in Kontakt bringen in Schritt (iii) des Verfahrens gemäß der vorliegenden Erfindung erfolgt im Fall, dass der Biokatalysator als immobilisiertes Enzym vorliegt, indem das Permeat (P1) an dem immobilisierten Enzym vorbeigeführt wird, sodass das Acrylamid mit dem Enzym reagieren kann ohne, dass das Enzym in das Permeat übergehen kann. Der entstehende Stoffstrom wird als Permeat (P2) bezeichnet beziehungsweise ist dem hierin beschriebenen Permeat (P2) gleichzusetzen.

In einer bevorzugten Ausführungsform liegt das immobilisierte Enzym als Teil eines Festbett- oder Wirbelschichtreaktors vor, in welchem das Permeat (P1) in Kontakt mit dem Biokatalysator gemäß Schritt (iii) gebracht und in das Permeat (P2) überführt wird. Vorzugsweise ändert sich der Gehalt an Acrylamid im räumlichen Verlauf des Reaktors, wobei der Gehalt an Acrylamid zu Beginn des Reaktors höher ist als am Ende des Reaktors. Besonders bevorzugt ist diese Ausführungsform unter Einsatz eines Festbettreaktors.

Im Kontext der vorliegenden Erfindung ist es bevorzugt, wenn das Enzym eine Größe zwischen 20 und 80 kDa, besonders bevorzugt zwischen 30 und 70 kDa und ganz besonders bevorzugt zwischen 40 und 60 kDa besitzt.

Im Kontext der vorliegenden Erfindung ist es besonders bevorzugt, wenn der Biokatalysator ein Größenverhältnis von 1: 10 bis 1: 1.000, besonders bevorzugt von 1: 10 bis 1: 100 zu den in der wässrigen Zubereitung aus Schritt (i) vorliegenden weiteren Komponenten (K1) besitzt.

In einer bevorzugten Ausführungsform beträgt das Größenverhältnis bezogen auf den Durchmesser von Acrylamid zu Biokatalysator 1: 5 bis 1: 5.000, weiterhin bevorzugt 1: 10 bis 1: 1.000, bevorzugt 1: 20 bis 1: 500 und weiterhin bevorzugt 1: 50 bis 1: 100.

Eine weitere bevorzugte Ausführungsform des Verfahrens gemäß der vorliegenden Erfindung betrifft ein Verfahren, wobei der Biokatalysator als (teil)aufgereinigtes Enzym und/oder als immobilisiertes Enzym vorliegt, welches eine Amidase, bevorzugt aus *Pseudonocardia thermophila, Sulfolobus solfataricus, Pyrococcus yayanosii* und *Sulfolobus tokodaii,* besonders bevorzugt aus *Pseudonocardia thermophila,* ist.

Weitere bevorzugt einzusetzende Enzyme zur Reduktion des Gehalts an Acrylamid sind Amidasen, wie im Stand der Technik beschrieben, vorzugsweise wie in den Anmeldungen WO 2004/083423 A1, WO 2006/040345 A2, WO 2012/032472 A2, EP 0272025 A2, WO 2021/1148508 und WO 2021/1148509 beschrieben.

Eine bevorzugte Ausführungsform betrifft ein Verfahren gemäß der vorliegenden Erfindung, wobei der Biokatalysator als (teil)aufgereinigtes Enzym und/oder als immobilisiertes Enzym vorliegt, welches eine Aminosäuresequenz mit einer Sequenzidentität von mindestens 60 %, 70 %, 80 %, 90 %, 91 %, 92 %, 93 %, 94 %, 95 %, 96 %, 97 %, 98 % oder 99 %, vorzugsweise von mindestens 90 %, 91 %, 92 %, 93 %, 94 %, 95 %, 96 %, 97 %, 98 % oder 99 %, zu der Gesamtlänge eines Enzyms mit einer Sequenz ausgewählt aus der Gruppe, bestehend aus den Sequenzen gemäß SEQ ID NO. 1 bis SEQ ID NO. 44, besonders bevorzugt ausgewählt aus der Gruppe bestehen aus den Sequenzen gemäß SEQ ID NO. 12 bis SEQ ID NO. 20 und ganz besonders bevorzugt ausgewählt aus der Gruppe bestehend aus den Sequenzen gemäß SEQ ID NO. 17 bis SEQ ID NO. 20 umfasst oder daraus besteht.

Wann immer die vorliegende Offenbarung auf Sequenzidentitäten von Aminosäuresequenzen in Form von Prozentangaben Bezug nimmt, beziehen sich diese Angaben auf Werte, wie sie unter Verwendung von EMBOSS Water Pairwise Sequence Alignments (Protein) für Aminosäuresequenzen berechnet werden können. Bei dem vom European Molecular Biology Laboratory (EMBL) European Bioinformatics Institute (EBI) zur Verfügung gestellten Tools für lokale Sequenzalignments wird ein modifizierter Smith-Waterman Algorithmus verwendet. Ferner wird hierbei bei der Durchführung des jeweiligen paarweisen Alignments zweier Sequenzen unter Verwendung des modifizierten Smith-Waterman Algorithmus auf die vom EMBL-EBI derzeit angegebenen Default Parameter Bezug genommen. Diese lauten (i) für Aminosäuresequenzen: Matrix = BLOSUM62, Gap open penalty = 10 und Gap extend penalty = 0,5 sowie (ii) für Nukleinsäuresequenzen: Matrix = DNAfull, Gap open penalty = 10 und Gap extend penalty = 0,5. Über die Default Parameter hinaus muss beim Alignment einer zu untersuchenden Sequenz ("Query Sequence", bei EMBOSS die erste Sequenz) mit einer Vergleichssequenz ("Subject Sequence", bei EMBOSS die zweite Sequenz) die Subject Sequence mindestens zu 93% über die Länge des einzelnen Alignments vertreten sein ("Sequence Coverage" mindestens 93%); Aligments mit einer niedrigeren Sequence Coverage der Subject Sequence sind für die Bestimmung der Sequenzidentität im Sinne dieser Anmeldung ausgeschlossen. Die Query Sequence kann jedoch über die Länge des Alignments hinaus länger sein, und die im Alignment vertretenen Sequenzen der Query Sequence können über oder unter 93% liegen.

Der Ausdruck "Sequenzidentität" ist daher im Kontext der vorliegenden Erfindung austauschbar mit "Sequenzhomologie" verwendbar. Dieser bezieht sich immer auf die Gesamtlänge eines erfindungsgemäßen Enzyms im Vergleich zu der Gesamtlänge eines Enzyms, zu dem die Sequenzidentität oder Sequenzhomologie bestimmt wird.

In einer weiteren bevorzugten Ausführungsform betrifft die vorliegende Erfindung ein Verfahren gemäß der vorliegenden Erfindung, wobei in Schritt (iii) das in Kontakt bringen des Permeats (P1) mit dem Biokatalysator durch Mischen erfolgt und anschließend diese Mischung in zwei Stoffströme aufgeteilt wird, um das Permeat (P2) und das Retentat (R2) zu erhalten, wobei das Permeat (P2) keinen Biokatalysator enthält.

Vorzugsweise erfolgt das in Kontakt bringen des Permeats (P1) mit dem Biokatalysator durch Mischen in einem Teilschritt (iii-1) und die anschließende Aufteilung der Mischung in zwei Stoffströme in einem Teilschritt (iii-2) über eine Membran (M2).

In einer weiteren bevorzugten Ausführungsform erfolgt der Schritt (iii-2) und Schritt (iv) gleichzeitig über eine Membran (M2).

In einer weiteren bevorzugten Ausführungsform erfolgt der Schritt (iii-2) und Schritt (iv) gleichzeitig über eine Membran (M1).

Eine weitere bevorzugte Ausführungsform des erfindungsgemäßen Verfahrens betrifft ein Verfahren, wobei das in Kontakt bringen des Biokatalysators in Schritt (iii) mit dem Permeat (P1) im Permeatraum eines ersten Membranmoduls stattfindet und das Zusammenführen des Permeats (P2) und des Retentats (R1) im Permeatraum eines zweiten Membranmoduls stattfindet und wobei das Retentat (R1) in den Permeatraum des zweiten Membranmoduls geführt wird. Es ist bevorzugt im Rahmen der vorliegenden Erfindung, dass die Verfahrensschritte iterativ, vorzugsweise kontinuierlich durchgeführt werden, bis eine gewünschte Reduktion an Acrylamid erreicht ist.

Eine weitere bevorzugte Ausführungsform des erfindungsgemäßen Verfahrens betrifft ein Verfahren, wobei das in Kontakt bringen des Biokatalysators in Schritt (iii) mit dem Permeat (P1) im Permeatraum eines ersten Membranmoduls (M1) stattfindet und das Zusammenführen des Permeats (P2) und des Retentats (R1) im Permeatraum eines zweiten Membranmoduls (M2) stattfindet und wobei das Retentat (R1) in den Permeatraum des zweiten Membranmoduls geführt wird. Es ist bevorzugt im Rahmen der vorliegenden Erfindung, dass die Verfahrensschritte iterativ, vorzugsweise kontinuierlich durchgeführt werden, bis eine gewünschte Reduktion an Acrylamid erreicht ist.

Eine weitere bevorzugte Ausführungsform des erfindungsgemäßen Verfahrens betrifft ein Verfahren, wobei das in Kontakt bringen des Biokatalysators in einem Teilschritt (iii-1) mit dem Permeat (P1) im Permeatraum eines ersten Membranmoduls (M1) stattfindet, in einem Teilschritt (iii-2) die Abtrennung des Biokatalysators aus dem Permeat (P2) mittels einer Membran (M2) erfolgt und in einem Schritt (iv) das Zusammenführen des Permeats (P2) und des Retentats (R1) im Permeatraum des zweiten Membranmoduls (M2) stattfindet und wobei das Retentat (R1) in den Permeatraum des zweiten Membranmoduls geführt wird. Es ist bevorzugt im Rahmen der vorliegenden Erfindung, dass die Verfahrensschritte iterativ, vorzugsweise kontinuierlich durchgeführt werden, bis eine gewünschte Reduktion an Acrylamid erreicht ist. Vorzugsweise erfolgen der Teilschritt (iii-2) und (iv) gleichzeitig.

Eine weitere bevorzugte Ausführungsform des erfindungsgemäßen Verfahrens betrifft ein Verfahren, wobei das in Kontakt bringen des Biokatalysators in einem Teilschritt (iii-1) mit dem Permeat (P1) im Permeatraum eines ersten Membranmoduls (M1) stattfindet, in einem Teilschritt (iii-2) die Abtrennung des Biokatalysators aus dem Permeat (P2) mittels derselben Membran (M1) erfolgt und in einem Schritt (iv) das Zusammenführen des Permeats (P2) und des Retentats (R1) im Retentatraum des ersten Membranmoduls (M1) stattfindet. Es ist bevorzugt im Rahmen der vorliegenden Erfindung, dass die Verfahrensschritte iterativ, vorzugsweise kontinuierlich durchgeführt werden, bis eine gewünschte Reduktion an Acrylamid erreicht ist. Vorzugsweise erfolgen der Teilschritt (iii)-1), (iii-2) und (iv) gleichzeitig.

Wiederum eine weitere bevorzugte Ausführungsform betrifft ein Verfahren, wobei das in Kontakt bringen des Permeats (P1) mit dem Biokatalysator über die Kontaktfläche einer Membran erfolgt und, wobei die Porengröße der Membran d₉₀ ≤ 100 kDa, bevorzugt d₉₀ ≤ 50 kDa, besonders bevorzugt d₉₀ ≤ 30 kDa beträgt und ganz besonders bevorzugt d₉₀ ≤ 10.

In einer weiteren bevorzugten Ausführungsform ist der Biokatalysator in der Matrix der Membran immobilisiert. Bevorzugt erfolgt die Reduktion des Gehalts an Acrylamid und die Entstehung eines Permeats (P2) dadurch, dass das Permeats (P1) an der Kontaktfläche der Membran vorbeifließt und das Acrylamid durch die Membran hindurch diffundiert und umgesetzt wird.

In einer bevorzugten Ausführungsform erfolgt die Umsetzung des Acrylamid durch den immobilisierten Biokatalysator bei Diffusion durch die Membran.

Eine weitere bevorzugte Ausführungsform betrifft ein Verfahren gemäß der vorliegenden Erfindung, wobei das Aufteilen der Stoffströme gemäß Schritt (ii) und das in Kontakt bringen des Permeats (P1) mit dem Biokatalysator gemäß Schritt (iii) gleichzeitig über die Kontaktfläche einer Membran erfolgt. Bevorzugt hierbei ist es, wenn mindestens ein Membranmodul verwendet wird und sich der Biokatalysator im Lumen beziehungsweise im Permeatraum dieses mindestens einen Membranmoduls befindet.

Eine weitere bevorzugte Ausführungsform betrifft ein Verfahren gemäß der vorliegenden Erfindung, wobei das Aufteilen der Stoffströme gemäß Schritt (ii) und das in Kontakt bringen des Permeats (P1) mit dem Biokatalysator gemäß Schritt (iii-1) gleichzeitig über die Kontaktfläche einer Membran (M1) erfolgt. Bevorzugt hierbei ist es, wenn mindestens ein Membranmodul (M1) verwendet wird und sich der Biokatalysator im Lumen beziehungsweise im Permeatraum dieses mindestens einen Membranmoduls befindet. Ebenfalls bevorzugt hierbei ist es, wenn die Aufteilung der Prozessströme gemäß Schritt (ii) und die Abtrennung des Biokatalysators gemäß Schritt (iii-2) und die Zusammenführung der Prozessströme gemäß Schritt (iv) kontinuierlich stattfinden und sich der Biokatalysator im Lumen beziehungsweise im Permeatraum dieses mindestens einen Membranmoduls (M1) befindet.

Bevorzugt im Kontext der vorliegenden Erfindung ist es, wenn zusätzlich das in Kontakt bringen des Permeats (P1) mit dem Biokatalysator und Erhalten eines Permeats (P2) mit reduziertem Gehalt an Acrylamid gemäß Schritt (iii) und das Zusammenführen des erhaltenen Permeats (P2) mit reduziertem Gehalt an Acrylamid und des Retentats (R1) gemäß Schritt (iv) gleichzeitig über die Kontaktfläche einer Membran erfolgt.

Bevorzugt im Kontext der vorliegenden Erfindung ist es, wenn zusätzlich das in Kontakt bringen des Permeats (P1) mit dem Biokatalysator und Erhalten eines Permeats (P2) mit reduziertem Gehalt an Acrylamid gemäß Schritt (iii) und das Zusammenführen des erhaltenen Permeats (P2) mit reduziertem Gehalt an Acrylamid und des Retentats (R1) gemäß Schritt (iv) gleichzeitig über die Kontaktfläche einer Membran (M2) erfolgt.

Besonders bevorzugt im Kontext der vorliegenden Erfindung ist es, wenn die Schritte (ii), (iii) und (iv) kontinuierlich über die Kontaktfläche einer einzigen Membran erfolgen. Vorzugsweise findet dabei das in Kontakt bringen des Biokatalysators mit dem Permeat (P1) und dem Erhalten von Permeat (P2) im Permeatraum eines einzigen Membranmoduls statt. Hierbei diffundiert das Acrylamid aus dem Retentatraum in den Permeatraum und kommt dort in Kontakt mit dem Biokatalysator. Die aus dem Permeatraum in den Retentatraum zurück diffundieren Stoffe (das sogenannte Permeat (P2)) enthalten einen reduzierten Gehalt an Acrylamid.

Besonders bevorzugt im Kontext der vorliegenden Erfindung ist es, wenn die Schritte (ii), (iii) und (iv) kontinuierlich über die Kontaktfläche einer einzigen Membran (M1) erfolgen und das in Kontakt bringen des Biokatalysators mit dem Permeat (P1) und dem Erhalten von Permeat P2 im Permeatraum des einzigen Membranmoduls (M1) stattfindet. Hierbei diffundiert das Acrylamid aus dem Retentatraum von Membran (M1) in den Permeatraum von Membran (M1) und kommt dort in Kontakt mit dem Biokatalysator. Die aus dem Permeatraum der Membran (M1) in deren Retentatraum zurück diffundieren Stoffe (das sogenannte Permeat (P2)) enthalten einen reduzierten Gehalt an Acrylamid.

Bei einer derartigen Verfahrensgestaltung ist es besonders bevorzugt, wenn ein Hohlfasermodul (hollow fibre membrane) als Membrangeometrie verwendet wird, wobei der Biokatalysator im Lumen bzw. im Permeatraum des Hohlfasermoduls vorliegt. Die wässrige Zubereitung wird durch das Hohlfasermodul geleitet und kommt mit dem Biokatalysator über eine Membran in Kontakt, wobei das Acrylamid durch die Membran diffundiert und von dem Biokatalysator umgesetzt wird. Das Permeat (P1) und das Retentat (R1) befinden sich räumlich zu Beginn des Hohlfasermoduls und das Permeat (P2) am Ende des Hohlfasermoduls. Des Weiteren sind die Flachmembran (flat sheet membrane) oder Laminar Stacks eine bevorzugte Membrangeometrie bei der Anwendung einer derartigen Verfahrensgestaltung.

Wiederum eine weitere bevorzugte Ausführungsform betrifft ein Verfahren gemäß der vorliegenden Erfindung, wobei die in Schritt (iv) erhaltene wässrige Zubereitung mit reduziertem Gehalt an Acrylamid im Vergleich zur in Schritt (i) bereitgestellten wässrigen Zubereitung einen um mindestens 20 Gew.-%, bevorzugt um mindestens 30 Gew.-%, weiterhin bevorzugt um mindestens 40 Gew.-%, wiederum bevorzugt um mindestens 50 Gew.-%, bevorzugt um mindestens 60 Gew.-%, weiterhin bevorzugt um mindestens 70 Gew.-%, bevorzugt von mindestens 80 Gew.-%, besonders bevorzugt von mindestens 90 Gew.-% reduzierten Gehalt an Acrylamid aufweist, bezogen auf das Gesamtgewicht der wässrigen Zubereitung,
und/oder
wobei die in Schritt (iv) erhaltene wässrige Zubereitung mit reduziertem Gehalt an Acrylamid einen Gehalt an Acrylamid von weniger als 2000 µg/kg, bevorzugt von weniger als 850 mg/kg und besonders bevorzugt von weniger als 500 µg/kg bezogen auf die Trockenmasse der Zubereitung aufweist.

Eine weitere bevorzugte Ausführungsform betrifft ein Verfahren gemäß der vorliegenden Erfindung, wobei die in Schritt (iv) erhaltene wässrige Zubereitung in Schritt (v) weiterverarbeitet wird und wobei die Weiterverarbeitung wenigstens einen Verfahrensschritt, ausgewählt aus der Gruppe bestehend aus Trocknen, Verdampfen, Konzentrieren, Gefriertrocknen, Wirbelschichttrocknen, Sprühtrocknen, Granulieren Zerkleinern und Filtrieren, umfasst.

Wiederum eine weitere bevorzugte Ausführungsform betrifft ein Verfahren gemäß der vorliegenden Erfindung, wobei die Schritte (ii) und (iii) wiederholt werden, vorzugsweise kontinuierlich.

Weiterhin hierin beschrieben ist eine Vorrichtung zum Entfernen von Acrylamid aus einer wässrigen Zubereitung, bestehend aus oder umfassend
(i) einen Vorlagenbehälter, in welchem die wässrige Zubereitung bereitgestellt wird,
(ii) ein erstes Membranmodul, vorzugsweise mit einer Membran (M1),
(iii) einen zweiten Vorlagenbehälter, in welchem der Biokatalysator bereitgestellt wird,
(iv) ein zweites Membranmodul, vorzugsweise mit einer Membran (M2),
wobei das erste Membranmodul und das zweite Membranmodul eine identische Porengröße aufweisen.

Eine bevorzugte Ausführungsform betrifft eine Vorrichtung, wobei beide Membranmodule eine Porengröße von d₉₀ ≤ 100 kDa, bevorzugt d₉₀ ≤ 50 kDa, besonders bevorzugt d₉₀ ≤ 30 kDa und ganz besonders bevorzugt d₉₀ ≤ 10 kDa besitzt.

Auch ist hierin eine Vorrichtung zum Entfernen von Acrylamid aus einer wässrigen Zubereitung beschrieben, bestehend aus oder umfassend
(i) einen Vorlagenbehälter, in welchem die wässrige Zubereitung bereitgestellt wird,
(ii) ein erstes Membranmodul, vorzugsweise mit einer Membran (M1),
(iii) einen zweiten Vorlagenbehälter, in welchem der Biokatalysator bereitgestellt wird,
(iv) ein zweites Membranmodul, vorzugsweise mit einer Membran (M2),
wobei das erste Membranmodul und das zweite Membranmodul unterschiedliche Porengrößen aufweisen.

Eine bevorzugte Ausführungsform betrifft eine Vorrichtung, wobei das erste der beiden Membranmodule eine Porengröße von d₉₀ ≤ 100 kDa, bevorzugt d₉₀ ≤ 50 kDa, besonders bevorzugt d₉₀ ≤ 30 kDa und ganz besonders bevorzugt d₉₀ ≤ 10 kDa besitzt und, wobei das zweite der beiden Membranmodule eine Porengröße von d₉₀ ≤ 100 kDa, bevorzugt d₉₀ ≤ 50 kDa, besonders bevorzugt d₉₀ ≤ 30 kDa und ganz besonders bevorzugt d₉₀ ≤ 10 kDa besitzt. Vorzugsweise ist die Porengröße des ersten der beiden Membranmodule kleiner als die Größe der Porengröße des zweiten der beiden Membranmodule. In einer besonders bevorzugten Ausführungsform sind die Porengrößen der beiden Membranmodule kleiner als der Biokatalysator.

Auch ist hierin eine Vorrichtung zum Entfernen von Acrylamid aus einer wässrigen Zubereitung beschrieben, bestehend aus oder umfassend
(i) einen Vorlagenbehälter, in welchem die wässrige Zubereitung bereitgestellt wird,
(ii) ein erstes Membranmodul, vorzugsweise mit einer Membran (M1),
(iii) einen zweiten Vorlagenbehälter, in welchem der Biokatalysator bereitgestellt wird,
wobei die Aufteilung und Zusammenführung der Prozessströme über das erste Membranmodul erfolgen.

Weiterhin hierin beschrieben ist eine Vorrichtung zum Entfernen von Acrylamid aus einer wässrigen Zubereitung, bestehen aus oder umfassend
(i) einen Vorlagebehälter, in welchem die wässrige Zubereitung bereitgestellt wird,
(ii) ein Membranmodul, vorzugsweise ein Hohlfasermembranmodul,
wobei das Membranmodul ein Lumen besitzt, in welchem ein Biokatalysator vorliegt.

Die Erfindung wird im Folgenden durch illustrative, nicht limitierende Beispiele charakterisiert.

### Beispiele

### Beispiel 1:

Rohkaffee der Sorte Brazil Grinder wurde auf einen Farbwert von 110 Skalenteilen (Neuhaus Neotec, Colortest II) geröstet. Der Acrylamidgehalt im gerösteten Kaffee wurde analysiert und betrug 560 µg/kg. Es wurden 40 kg Röstkaffee mit einer Extraktionstemperatur von 85 °C extrahiert, was eine Ausbeute von insgesamt 220 kg Kaffee-Extrakt ergab. Ein Teil des Kaffeeextraktes wurde für die nachfolgenden Versuche verwendet und ein Teil wurde für die Gefrierkonzentration eingesetzt, um das Kaffeekonzentrat herzustellen. Der unkonzentrierte Kaffeeextrakt hatte einen Trockenrückstand von 1,4 % und einen Acrylamidgehalt von 28 µg/L, und das Konzentrat einen Trockenrückstand von 27,5 % und einen Acrylamidgehalt von 630 µg/L.

Der unkonzentrierte Kaffeeextrakt mit einem Trockenrückstand von 1,4 Gew.-% wurde über eine Membran (Hohlfasermodul, Cut-Off: 10 kDA) aus Polyethersulfon in zwei Stoffströme Permeat (P1) und Retentat (R1) aufgeteilt. Das Permeat (P1) besaß einen Acrylamidgehalt von 23 µg/L, welcher auf 223 µg/L durch Zugabe von Acrylamid erhöht wurde, und wurde anschließend in einen Vorlagebehälter geführt und mit 6500 U/L Amidase behandelt. Die Reaktion wurde bei 70 °C durchgeführt. Anschließend wurde das Permeat (P1) mit der Amidase über eine weitere Membran bei einer Flussrate von 2 mL/min in zwei Stoffströme aufgeteilt. Das erhaltene Permeat (P2) enthält keine eingesetzte Amidase.

Die Reduktion an Acrylamid im Permeat (P2) errechnet sich aus den enzymbehandelten Proben verglichen zur Referenz ((P1) mit 200 µg/L Acrylamid versetzt, ohne Enzymzugabe). Die Acrylamidreduktion liegt für eine Prozesszeit von 30 - 300 Minuten konstant bei 80 - 90 %.

Die Begriffe Trockenrückstand und Feststoffgehalt haben die gleiche Bedeutung.

### Beispiel 2:

Der Versuchsaufbau aus Beispiel 1 wurde für eine kontinuierliche Fahrweise genutzt und das erhaltene Permeat (P2) zu dem Ausgangskaffeeextrakt zurückgeführt. Die Acrylamidreduktion nach 60 -120 Minuten lag bei 84 - 93 %.

### Beispiel 3:

In einem weiteren Versuch wurde der in Beispiel 1 hergestellte Kaffeeextrakt ohne vorherige Auftrennung in einem Diffusionsmembranreaktor behandelt. Hierfür wurde ein Hohlfasermodul mit einem Cut-Off von 10 kDa und einer Membranfläche von 155 cm² verwendet, durch welches der Kaffeeextrakt (versetzt mit 300 µg/L Acrylamid) bei 50 °C zirkuliert wurde. Auf der Permeatseite des Hohlfasermoduls wurde eine Enzymlösung mit Amidase in einer Konzentration von 940 U/L gegeben. Diese wurde nicht im Kreis gepumpt. Das Acrylamid diffundiert durch die Membran und wird von der Amidase abgebaut. Die Acrylamidreduktion nach einer Reaktionszeit von 200 Minuten betrug 35 %. Die Acrylamidreduktion konnte bei Verwendung einer größeren Membranfläche durch Hintereinanderschaltung von zwei Modulen und Erhöhung der Enzymkonzentration auf der Permeatseite auf 8.000 U/L auf einen Wert von 90 % gesteigert werden.

### Beispiel 4: Herstellung von Kaffeeextrakt

Rohkaffee der Sorte Vietnam Robusta wurde auf einen Farbwert von 102 Skalenteilen (Neuhaus Neotec, Colortest II) geröstet. Der Acrylamidgehalt im gerösteten Kaffee wurde analysiert und betrug 360 µg/kg. Es wurden 20 kg Röstkaffee mit einer Extraktionstemperatur von 110 °C extrahiert, was eine Ausbeute von insgesamt 80 kg Kaffee-Extrakt ergab. Der Kaffeeextrakt hatte einen Trockenrückstand (Feststoffgehalt) von 4,5 % und einen Acrylamidgehalt von 50 µg/L.

### Beispiel 5: Herstellung eines KEF (Kaffee-Extrakt-Filtrats); Permeat (P1)

### Beispiel 5.1: Herstellung von KEF im Labormaßstab

500 mL Kaffee-Extrakt, entsprechend Beispiel 4, wurden in einer Querstromfiltration über ein Kapillarmodul der Firma Repligen mit Fasern aus mPES, einem Cut-Off von 10 kDa und einer Filterfläche von 75 cm² filtriert und ein Permeat (P1) und ein Retentat (R1) erhalten. Der Transmembrandruck betrug 1,3 - 1,7 bar. Innerhalb von 16 h wurden 400 ml Permeat (P1) gewonnen. Der Versuch fand bei Raumtemperatur statt.

### Beispiel 5.2: Herstellung von KEF im Pilotmaßstab

### Herstellung mit Membranmodul

80 L Kaffee-Extrakt mit 4,5% Feststoffgehalt entsprechend Beispiel 4 wurden in einer Querstromfiltration über ein Membran-Modul der Firma Microdyn-Nadir mit einer Fläche von 5 m³ filtriert (FS10-FC-FUS0181). Das Membranmaterial bestand aus PES mit einem Cut-Off von 10 kDa. Die einzelnen Fasern hatten einen Innendurchmesser von 0,8 mm. Bei einer Temperatur von 50 °C, einer Überströmrate von 1 m/s und einem TMP von 2 bar wurden 70 L Permeat (P1) (filtrierter Kaffeeextrakt) innerhalb von 25 min gewonnen.

### Herstellung mit Keramikmodul

61 L Kaffee-Extrakt mit 4,5% Feststoffgehalt entsprechend Beispiel 4 wurden in einer Querstromfiltration über ein Keramik-Modul der Firma Atech mit einer Fläche von 0,236 m³ filtriert. Das keramische Membranmaterial hat einen Cut-Off von 25 kDa. Die einzelnen Kanäle hatten einen Innendurchmesser von 3,3 mm. Bei einer Temperatur von 50 °C, einer Überströmrate von 5 m/s und einem TMP von 1,5 bar wurden 20,5 L Permeat (P1) (filtrierter Kaffeeextrakt) innerhalb von 48 min gewonnen.

### Beispiel 6: Enzymbehandlung von Permeat P1, Abtrennung Biokatalysator

### Beispiel 6.1: Abtrennung des Biokatalysators über UF

1 L filtrierter KEF (Permeat (P1)) entsprechend Beispiel 5 wurde mit 1 mM Acrylamid versetzt, um die Analytik zu vereinfachen. Es wurden 1000 U eines flüssigen Amidase-Präparats zugegeben, gemischt und für 2 h bei 40 °C inkubiert. Die Acrylamidkonzentration verringerte sich um 72% im Vergleich zum unbehandelten Kaffeefiltrat (Permeat (P1)).

Zur Abtrennung des Enzyms wurde eine Querstromfiltration durchgeführt. Dazu wurde ein Membranmodul mit einer Cellulose basierten Membran mit einer Fläche von 200 cm² und einer Porengröße von 10 kDa genutzt. Das behandelte KEF wurde von 1 L auf etwa 50 ml eingeengt. Es wurden etwa 950 ml behandeltes enzymfreies KEF (Permeat (P2)) erhalten.

### Beispiel 6.2: Kontinuierliche Enzymbehandlung und Abtrennung von behandeltem Permeat (P2)

Der Aufbau zur kontinuierlichen Enzymbehandlung bestand aus einem Reaktionsbehälter, einem Vorlagenbehälter, einer Peristaltikpumpe, einem Membranmodul und einem Permeatbehälter, welche über Schläuche miteinander verbunden waren. Der Vorlagenbehälter war mit KEF (Permeat (P1) entsprechend Beispiel 5) gefüllt, welches mit 1 mM Acrylamid angereichert wurde, um die Analytik zu vereinfachen. Der Reaktionsbehälter bestand aus einem Gefäß in welches 75 ml KEF (Permeat (P1) entsprechend Beispiel 5) vorgelegt und mit 320 U eines flüssigen Amidase-Präparats versetzt wurde. Der Reaktionsansatz wurde im Kreis über das Membranmodul (200 cm², 10 kDa Cellulose basiertes Membranmaterial) und anschließend zurück in den Reaktionsbehälter gepumpt. Der Permeatfluss des Membranmoduls wurde über die Pumpleistung reguliert und auf einen Wert von 1 ml/min eingestellt. Das Permeat wurde im Permeatbehälter gesammelt. Das Volumen des Reaktionsansatzes wurde konstant gehalten, indem filtrierter Kaffee-Extrakt aus dem Vorlagenbehälter in den Reaktionsbehälter gefördert wurde. Aus Größe des Reaktionsansatzes und der Permeatflussrate ergab sich eine mittlere Verweildauer für das KEF von 75 min. Nach Gleichgewichtseinstellung ergibt sich im Permeat eine Acrylamidreduktion in Höhe von 74%.

### Beispiel 6.3: Kontinuierliche Enzymbehandlung mittels Immobilisat

Ein poröser Enzym-Träger mit Partikelgrößen von 300 - 700 µm und Poren im Größenbereich von 1200-1800 Å wurde mit Enzym kovalent beladen. Die spezifische Aktivität des so erhaltenen Enzym-Immobilisats betrug 45 U/g.

5 g des Immobilisats wurden in eine Chromatographie-Säule mit Siebboden und 1,6 cm Innendurchmesser gepackt. Die Vorlage mit KEF (Permeat (P1) entsprechend Beispiel 5), angereichert mit 1 mM Acrylamid, und die mit Immobilisat gefüllte Säule wurden auf 40°C temperiert. KEF (Permeat (P1) entsprechend Beispiel 5) wurde mit einer Flussrate von 2 ml/min kontinuierlich über das Säulenbett geleitet und fraktioniert als Permeat (P2) aufgefangen. Die Konzentration an Acrylamid im Durchfluss der Säule war um 87% reduziert.

### Beispiel 7: Acrylamid-Abbau als kombiniertes 2-stufiges Membranverfahren

Die kombinierte Filtration des Kaffee-Extrakts, der enzymatischen Behandlung mit Amidase und der Abtrennung des Biokatalysators erfolgt durch die apparative Verschaltung von zwei Querstromfiltrationsapparaturen. Das Vorlagengefäß 1 von Apparatur 1 wird mit 10 L eines mit Acrylamid auf 1 mM angereicherten Kaffee-Extrakts (Permeat (P1) entsprechend Beispiel 4) gefüllt. Das Vorlagengefäß 1 wird über eine Pumpe an ein Kapillarmodul 1 der Firma Microdyn-Nadir mit 0,25 m² Filterfläche bestehend aus einer PES-Membran mit 30 kDa Poren angeschlossen. Das Retentat (R1) wird nach Verlassen des Kapillarmoduls 1 wieder in Vorlagengefäß 1 zurückgeführt. Der Permeatauslass von Kapillarmodul 1 mündet in das Vorlagengefäß 2 von Apparatur 2. Im Vorlagengefäß 2 werden 2000 U eines Amidasepräparats vorgelegt. Vorlagengefäß 2 ist über eine 2. Pumpe mit einem Kapillarmodul 2, identisch zu Kapillarmodul 1, verbunden, dessen Retentat in Vorlagengefäß 2 zurückgeführt wurde. Der Permeat-Auslass von Kapillarmodul 2 ist mit Vorlagengefäß 1 von Apparatur 1 verbunden.

Zunächst wird Apparatur 1 mit einer Flussrate von 11 L/min und einem TMP von 1,5 bar gestartet, wodurch Filtrat gebildet wird, welches im Vorlagengefäß 2 mündet. Nach Erreichen des Sollvolumens im Vorlagengefäß 2 wird Apparatur 2 mit einer Flussrate von 5 L/min gestartet. Der TMP von Apparatur 2 wird so geregelt (1 - 1,5 bar), dass der Permeatfluss von Apparatur 2 dem von Apparatur 1 gleicht und somit das Sollvolumen in Vorlagengefäß 2 konstant bleibt. Die Acrylamidkonzentration des Kaffee-Extrakts in Vorlagengefäß 1 wird kontinuierlich gemessen. Die Acrylamidkonzentration ist nach 2 h um bis zu 71% reduziert. Zum Beenden der Behandlung wird Apparatur 1 gestoppt und das Volumen in Apparatur 2 auf das Totvolumen von Apparatur 2 in Höhe von 0,4 L eingeengt.

### Beispiel 8: Acrylamid-Abbau als Diffusionsmembranreaktor (DMR-)-Verfahren

### Beispiel 8.1: Batch-Verfahren Labormaßstab

Für die direkte Behandlung von Kaffee-Extrakt entsprechend Beispiel 4 werden 1 L Kaffee-Extrakt entsprechend Beispiel 4 mit 1 mM Acrylamid versetzt und bei 40 °C im Kreis über ein Kapillarmodul der Firma Repligen mit Fasern aus mPES, einem Cut-off von 10 kDa und einer Filterfläche von 75 cm² gepumpt (Membran (M1)). Auf der geschlossenen Permeatseite befindet sich ein Amidase-Enzympräparat mit einer Aktivität von 500 U. Die Abnahme der Acrylamidkonzentration im Kaffee-Extrakt wird über die Zeit verfolgt. Nach 2 h ist der Acrylamidgehalt um 32% reduziert.

### Beispiel 8.2: Kontinuierliches Verfahren im Labormaßstab

Für eine kontinuierliche Behandlung wurden in einem weiteren Versuch die Retentaträume von 3 Kapillarmodulen (Repligen, mPES, 10 kDa, 75 cm²) nacheinander über Schläuche verbunden. Die Permeaträume der 3 Kapillarmodule waren ebenfalls verbunden und mit 600 U eines Amidase-Präparats gefüllt. Bei einer Temperatur von 40 °C wurde Kaffee-Extrakt entsprechend Beispiel 4, welcher mit 1 mM Acrylamid versetzt wurde, mit einer Flussrate von 0,6 ml/min über die Retentatseite der Kapillarmodule gepumpt und fraktioniert gesammelt. Die Acrylamidreduktion im Kaffee-Extrakt am Auslass des 3. Kapillarmoduls betrug 49%.

### Beispiel 8.3: Batch-Verfahren im Pilotmaßstab

32 L Kaffee-Extrakt entsprechend Beispiel 4 angereichert mit 3 µM Acrylamid wurden bei 50 °C über ein Membranmodul der Firma Microdyn-Nadir mit einer Fläche von 5 m³ mit einer Geschwindigkeit von 5,3 L/min gefördert (FS10-FC-FUS0181). Das Membranmaterial bestand aus PES mit einem Cut-off von 10 kDa. Die beiden Auslässe des Permeatraums waren mit einem Reservoir verbunden und mit 128 kU eines flüssigen Amidase-Präparats gefüllt. Die Flüssigkeit wurde über eine Peristaltikpumpe in Bewegung gehalten. Die Abnahme der Acrylamidkonzentration wurde über die Zeit verfolgt. Nach 90 min war die Acrylamidkonzentration um 92% reduziert.

### Beispiel 9: Herstellung eines acrylamidreduzierten Löskaffee-Produkts

### Beispiel 9.1: Herstellung mittels zweistufigem Membranverfahren

20 kg Kaffee der Sorte Brazil NY 17/18 wurde auf einen Farbwert von 110 (Neuhaus Neotec, Colortest II) geröstet und bei 110 °C extrahiert. Es wurden 100 kg Kaffee-Extrakt mit einem Feststoffgehalt von 4,1% und einem Acrylamidgehalt von 50 µg/L erhalten.

32 kg des Extrakts wurden in einer Querstromfiltrationsanlage über ein keramisches Modul der Firma Atech mit einem MWCO von 25 kDa, einer Überströmrate von 5 m/s und einem Transmembrandruck von 3,8 bar bei 50 °C filtriert. Es wurden 18,8 kg KEF (Permeat (P1)) gewonnen, welches mit 2000 U/L Amidase versetzt und für 1 h bei 50 °C inkubiert wurden. Von den ursprünglichen 32 kg des Extraktes sind 11 kg des Retentats (R1) verblieben und zur Seite gelegt worden. Das nach der Reaktion mit der Amidase erhaltene Permeat (P2) wurde anschließend einer zweiten Querstromfiltration zugeführt, um das Enzym abzutrennen. Es wurden 16,3 kg Permeat (P2) ohne Biokatalysator erhalten. Das behandelte KEF (Permeat (P2)) wurde mit 11 kg des Retentats (R1) vereint. Die so erhaltene Mischung aus konzentriertem Kaffeeextrakt (Retentat (R1) der ersten Querstromfiltration) und behandeltem Kaffeeextrakt-Filtrat (Permeat (P2) der zweiten Querstromfiltration) wurden in flache Schalen überführt, bei -40 °C eingefroren und im Anschluss in einer Gefriertrocknungsanlage für 29 h gefriergetrocknet. Es wurden 140 g Löskaffeeprodukt erhalten.

In gleicher Weise wurden 3,6 kg unbehandelter Kaffee-Extrakt, wie zu Beginn dieses Beispiels hergestellt, gefriergetrocknet.

Der Acrylamidgehalt des behandelten und des unbehandelten Kaffee-Extrakts wurden bestimmt. Demnach hat die behandelte Probe einen um 29% verringerten Acrylamidgehalt im Vergleich zur unbehandelten Kontrollprobe.

| Probe | Acrylamid [µg/kg] | Massenverlust [%] | Acrylamidreduktion [%] |
|---|---|---|---|
| Unbehandelte Probe | 1100 | 3,1 | - |
| Enzymbehandelte Probe | 780 | 3,5 | 29 |

### Beispiel 9.2: Herstellung mittels Diffusionsmembranreaktor (DMR)

Rohkaffee einer Arabica-Mischung wurden auf einen Farbwert von 110 geröstet. 40 kg des Kaffees wurden bei 85 °C extrahiert. Es wurden 200 kg Kaffeeextrakt gewonnen. Anschließend erfolgte die Konzentrierung auf einen Feststoffgehalt von 12,5 % mittels Gefrierkonzentrierung.

Der konzentrierte Extrakt hatte einen Acrylamidgehalt von 120 µg/L. Die Behandlung des konzentrierten Extrakts erfolgte über einen Diffusionsmembranreaktor bestehend aus einer Querstromfiltrationsanlage mit einem angeschlossenen Hohlfasermodul der Firma Microdyn-Nadir mit einer Filterfläche von 5 m² und einem MWCO von 10 kDa. Im Permeatraum (P1) des Moduls befanden sich 217,6 kU Amidase.

32 L des Extrakts wurden drucklos bei 50 °C für 2 h über das Hohlfasermodul im Kreis gepumpt. Anschließend wurden 0,6 kg des behandelten KEF (Permeat (P2)) in flache Schalen überführt, bei -40 °C eingefroren und gefriergetrocknet.

In gleicher Weise wurden 0,6 kg konzentrierter Extrakt gefriergetrocknet, welcher keiner Enzymbehandlung unterzogen wurde. Die Acrylamid-Gehalte der beiden Löskaffeeprodukte wurden untersucht und verglichen. Das Produkt aus behandeltem Extrakt hat ein um 34 % reduzierten Acrylamid-Gehalt.

| Probe | Acrylamid [µg/kg] | Massenverlust [%] | Acrylamidreduktion [%] |
|---|---|---|---|
| Unbehandelte Probe | 750 | 4,53 | - |
| Enzymbehandelte Probe | 100 | 4,97 | 87 |

## Patentansprüche

1. Verfahren zum Entfernen oder Umsetzen von Acrylamid aus einer wässrigen Zubereitung zur Herstellung eines Endprodukts mit verringertem Gehalt an Acrylamid, umfassend die folgenden Schritte:
(i) Bereitstellen einer wässrigen Zubereitung enthaltend Acrylamid und weitere Komponenten als Ausgangsprodukt zur Herstellung eines Endprodukts mit verringertem Gehalt an Acrylamid;
(ii) Aufteilen der wässrigen Zubereitung in zwei Stoffströme, um ein Retentat (R1) und ein Permeat (P1) zu erhalten, wobei das Retentat (R1) die weiteren Komponenten (K1) enthält, und wobei das Permeat (P1) Acrylamid sowie vorzugsweise keine oder einen geringen Anteil an weiteren Komponenten (K2) enthält;
(iii) in Kontakt bringen des Permeats (P1) mit einem Acrylamid reduzierenden Biokatalysator und Erhalten eines Permeats (P2) mit reduziertem Gehalt an Acrylamid;
(iv) Zusammenführen des erhaltenen Permeats (P2) mit reduziertem Gehalt an Acrylamid und des Retentats (R1) aus Schritt (ii) und Erhalten einer wässrigen Zubereitung mit reduziertem Gehalt an Acrylamid, wobei das Permeat (P2) keinen Biokatalysator enthält;
(v) optional, Weiterverarbeiten der wässrigen Zubereitung mit reduziertem Gehalt an Acrylamid aus Schritt (iv), um ein Endprodukt zu erhalten.

2. Verfahren nach Anspruch 1, wobei das Aufteilen der Stoffströme in Schritt (ii) als Filtration durchgeführt wird, vorzugweise als Membranfiltration.

3. Verfahren nach einem der Ansprüche 1 oder 2, wobei das Aufteilen der Stoffströme in Schritt (ii) als Membranfiltration durchgeführt wird und die Porengröße der Membran d₉₀ ≤ 100 kDa, bevorzugt d₉₀ ≤ 50 kDa, besonders bevorzugt d₉₀ ≤ 30 kDa beträgt
und/oder
wobei der Transmembrandruck zwischen 0,01 mbar und 10 bar, bevorzugt zwischen 0,1 mbar und 7,5 bar, weiterhin bevorzugt zwischen 1 mbar und 5 bar besonders bevorzugt zwischen 500 mbar und 2,5 bar liegt und/oder gehalten wird.

4. Verfahren nach einem der vorangehenden Ansprüche, wobei das Endprodukt ein dem Genuss, der Ernährung dienenden oder kosmetischen Endprodukt ist, und bevorzugt ausgewählt ist aus der Gruppe umfassend gebratene oder frittierte Kartoffelprodukte, Maisprodukte, Kaffeeprodukte, Kaffeeersatzprodukte, Snacks, Weizenprodukte, Kosmetika, Feingebäck wie Plätzchen, Kekse, Zwieback, Getreideriegel, Scones, Eiswaffeln, Waffeln, Crumpets, Lebkuchen, Knäckebrot und Brotersatzprodukte, Nudeln, Reis, Fischerzeugnisse, Fleischerzeugnisse, Cerealien, Bier oder Beikost für Kinder und Säuglinge.

5. Verfahren nach einem der vorangehenden Ansprüche, wobei der Biokatalysator als lebender Mikroorganismus und/oder als inaktivierter Mikroorganismus und/oder als Zelllysat und/oder als (teil)aufgereinigtes Enzym und/oder als immobilisiertes Enzym vorliegt.

6. Verfahren nach einem der vorangehenden Ansprüche, wobei der Biokatalysator als (teil)aufgereinigtes Enzym und/oder als immobilisiertes Enzym vorliegt, welches eine Amidase ist.

7. Verfahren nach einem der vorangehenden Ansprüche, wobei der Biokatalysator als (teil)aufgereinigtes Enzym und/oder als immobilisiertes Enzym vorliegt, welches eine Aminosäuresequenz mit einer Sequenzidentität von mindestens 60%, 70%, 80%, 90 %, 91 %, 92 %, 93 %, 94 %, 95 %, 96 %, 97 %, 98 % oder 99 %, vorzugsweise von mindestens 90 %, 91 %, 92 %, 93 %, 94 %, 95 %, 96 %, 97 %, 98 % oder 99 %, zu der Gesamtlänge eines Enzyms mit einer Sequenz ausgewählt aus der Gruppe, bestehend aus den Sequenzen gemäß SEQ ID NO. 1 bis SEQ ID NO. 44, umfasst oder daraus besteht.

8. Verfahren nach einem der vorangehenden Ansprüche, wobei in Schritt (iii) das in Kontakt bringen des Permeats (P1) mit dem Biokatalysator durch Mischen erfolgt und anschließend diese Mischung in zwei Stoffströme aufgeteilt wird, um das Permeat (P2) zu erhalten, wobei das Permeat (P2) keinen Biokatalysator enthält.

9. Verfahren nach einem der vorangehenden Ansprüche, wobei das in Kontakt bringen des Permeats (P1) mit dem Biokatalysator über die Kontaktfläche einer Membran erfolgt.

10. Verfahren nach einem der vorangehenden Ansprüche, wobei Schritt (iii) die folgenden Teilschritte umfasst:
(iii-1) in Kontakt bringen des Permeats (P1) mit einem Acrylamid reduzierenden Biokatalysator und Erhalten eines Permeats (P2) mit reduziertem Gehalt an Acrylamid wobei das Permeat (P2) den Biokatalysator umfasst;
(iii-2) Abtrennung des Biokatalysators aus dem Permeat (P2) aus Schritt (iii-1) mittels Separation über eine Membran (M2) und Erhalt eines Retentats (R2), das den Biokatalysator beinhaltet und Erhalt des Permeats (P2) mit reduziertem Gehalt an Acrylamid wobei das Permeat (P2) keinen Biokatalysator umfasst.

11. Verfahren nach einem der vorangehenden Ansprüche, vorzugsweise nach Anspruch 10, wobei das Aufteilen der wässrigen Zubereitung in zwei Stoffströme in Schritt (ii), und die Abtrennung des Biokatalysators vor Schritt (iv) über eine einzige Membran (M1) erfolgen.

12. Verfahren nach einem der Ansprüche 10 oder 11, wobei die Schritte (ii) und (iii-2) des erfindungsgemäßen Verfahrens über eine einzige Membran (M1) erfolgen.

13. Verfahren nach einem der Ansprüche 10 bis 12, wobei die Porengrößen der Membran (M1) kleiner oder gleich der Porengröße der Membran (M2) ist, und die Porengrößen von Membran (M1) und Membran (M2) jeweils kleiner als der Biokatalysator sind.

14. Verfahren nach einem der Ansprüche 10 bis 13, wobei die Porengröße der Membran, vorzugsweise der Membran (M1), d₉₀ ≤ 100 kDa, bevorzugt d₉₀ ≤ 50 kDa, besonders bevorzugt d₉₀ ≤ 30 kDa beträgt, ganz besonders bevorzugt d₉₀ ≤ 10 kDa
und/oder
wobei der Transmembrandruck zwischen 0,01 mbar und 10 bar, bevorzugt zwischen 0,1 mbar und 7,5 bar, weiterhin bevorzugt zwischen 1 mbar und 5 bar besonders bevorzugt zwischen 500 mbar und 2,5 bar liegt und/oder gehalten wird.

15. Verfahren nach einem der vorangehenden Ansprüche, wobei die in Schritt (iv) erhaltene wässrige Zubereitung mit reduziertem Gehalt an Acrylamid im Vergleich zur in Schritt (i) bereitgestellten wässrigen Zubereitung einen um mindestens 20 Gew.-%, bevorzugt um mindestens 30 Gew.-%, weiterhin bevorzugt um mindestens 40 Gew.-%, wiederum bevorzugt um mindestens 50 Gew.-%, bevorzugt um mindestens 60 Gew.-%, weiterhin bevorzugt um mindestens 70 Gew.-%, bevorzugt von mindestens 80 Gew.-%, besonders bevorzugt von mindestens 90 Gew.-% reduzierten Gehalt an Acrylamid aufweist, bezogen auf das Gesamtgewicht der wässrigen Zubereitung,
und/oder
wobei die in Schritt (iv) erhaltene wässrige Zubereitung mit reduziertem Gehalt an Acrylamid einen Gehalt an Acrylamid von weniger als 2000 µg/kg, bevorzugt von weniger als 850 mg/kg und besonders bevorzugt von weniger als 500 µg/kg bezogen auf die Trockenmasse der Zubereitung aufweist
und/oder
wobei die in Schritt (iv) erhaltene wässrige Zubereitung in Schritt (v) weiterverarbeitet wird und wobei die Weiterverarbeitung wenigstens einen Verfahrensschritt, ausgewählt aus der Gruppe bestehend aus Trocknen, Verdampfen, Konzentrieren, Gefriertrocknen, Wirbelschichttrocknen, Sprühtrocknen, Granulieren Zerkleinern und Filtrieren, umfasst.

## Claims

1. A method for removing or reacting acrylamide from an aqueous preparation to produce a final product having a reduced acrylamide content, comprising the following steps:
(i) Providing an aqueous preparation containing acrylamide and other components as a starting product for the manufacturing of a final product with a reduced acrylamide content;
(ii) dividing the aqueous preparation into two material streams to obtain a retentate (R1) and a permeate (P1), wherein the retentate (R1) contains the further components (K1), and wherein the permeate (P1) contains acrylamide and preferably no or a small proportion of further components (K2);
(iii) bringing the permeate (P1) into contact with an acrylamide-reducing biocatalyst and obtaining a permeate (P2) with a reduced acrylamide content;
(iv) combining the resulting permeate (P2) having a reduced content of acrylamide and the retentate (R1) from step (ii) and obtaining an aqueous preparation having a reduced content of acrylamide, wherein the permeate (P2) does not contain the biocatalyst;
(v) optionally, further processing the aqueous preparation with reduced content of acrylamide from step (iv) to obtain a final product.

2. The method according to claim 1, wherein the separation of the material streams in step (ii) is carried out as filtration, preferably as membrane filtration.

3. Method according to any one of claims 1 or 2, wherein the separation of the material streams in step (ii) is carried out as membrane filtration and the pore size of the membrane is d₉₀≤ 100 kDa, preferably d₉₀≤ 50 kDa, particularly preferably d₉₀≤ 30 kDa
and/or
wherein the transmembrane pressure is and/or is maintained between 0.01 mbar and 10 bar, preferably between 0.1 mbar and 7.5 bar, more preferably between 1 mbar and 5 bar, especially preferably between 500 mbar and 2.5 bar.

4. The method according to any one of the preceding claims, wherein the end product is a savory, nutritional or cosmetic end product, and is preferably selected from the group comprising fried or deep-fried potato products, corn products, coffee products, coffee substitutes, snacks, wheat products, cosmetics, pastries such as cookies, biscuits, rusks, cereal bars, scones, ice cream wafers, waffles, crumpets, lemons, and other baked goods, snacks, wheat products, cosmetics, cookies such as cookies, cookies, rusks, cereal bars, scones, ice cream cones, waffles, crumpets, gingerbread, crispbread and bread substitutes, pasta, rice, fish products, meat products, cereals, beer or baby food for children and infants.

5. The method according to any one of the preceding claims, wherein the biocatalyst is present as a living microorganism and/or as an inactivated microorganism and/or as a cell lysate and/or as a (partially) purified enzyme and/or as an immobilized enzyme.

6. The method according to any one of the preceding claims, wherein the biocatalyst is present as a (partially) purified enzyme and/or as an immobilized enzyme which is an amidase.

7. The method according to any one of the preceding claims, wherein the biocatalyst is present as a (partially) purified enzyme and/or as an immobilized enzyme which has an amino acid sequence with a sequence identity of at least 60%, 70%, 80%, 90%, 91%, 92%, 93%, 94%, 95%, 96%, 97%, 98% or 99%, preferably of at least 90%, 91%, 92%, 93%, 94%, 95%, 96%, 97%, 98% or 99%, to the total length of an enzyme having a sequence selected from the group consisting of the sequences according to SEQ ID NO. 1 to SEQ ID NO. 44, or consists thereof.

8. The method according to any one of the preceding claims, wherein in step (iii) the permeate (P1) is brought into contact with the biocatalyst by mixing and subsequently this mixture is divided into two material streams to obtain the permeate (P2), wherein the permeate (P2) does not contain a biocatalyst.

9. The method according to one of the preceding claims, wherein the permeate (P1) is brought into contact with the biocatalyst via the contact surface of a membrane.

10. The method according to any one of the preceding claims, wherein step (iii) comprises the following substeps:
(iii-1) contacting the permeate (P1) with an acrylamide-reducing biocatalyst and obtaining a permeate (P2) having a reduced content of acrylamide, the permeate (P2) comprising the biocatalyst;
(iii-2) separating the biocatalyst from the permeate (P2) from step (iii-1) by means of separation over a membrane (M2) and obtaining a retentate (R2) containing the biocatalyst and obtaining the permeate (P2) with a reduced content of acrylamide, wherein the permeate (P2) does not comprise any biocatalyst.

11. The method according to any one of the preceding claims, preferably according to claim 10, wherein the separation of the aqueous preparation into two material streams in step (ii), and the separation of the biocatalyst before step (iv) are carried out via a single membrane (M1).

12. The method according to any one of claims 10 or 11, wherein steps (ii) and (iii-2) of the method according to the invention are carried out over a single membrane (M1).

13. The method according to any one of claims 10 to 12, wherein the pore sizes of the membrane (M1) are smaller than or equal to the pore size of the membrane (M2), and the pore sizes of the membrane (M1) and the membrane (M2) are each smaller than the biocatalyst.

14. The method according to any one of claims 10 to 13, wherein the pore size of the membrane, preferably of the membrane (M1), is d₉₀≤ 100 kDa, preferably d₉₀≤ 50 kDa, more preferably d₉₀≤ 30 kDa, most preferably d₉₀≤ 10 kDa
and/or
wherein the transmembrane pressure is and/or is maintained between 0.01 mbar and 10 bar, preferably between 0.1 mbar and 7.5 bar, more preferably between 1 mbar and 5 bar, particularly preferably between 500 mbar and 2.5 bar.

15. The method according to any one of the preceding claims, wherein the aqueous preparation with reduced acrylamide content obtained in step (iv) has, compared to the aqueous preparation provided in step (i), an acrylamide content of at least 20% by weight, preferably of at least 30% by weight, further preferably of at least 40% by weight, again preferably of at least 50% by weight, further preferably of at least 60% by weight, more preferably of at least 80% by weight.% by weight, again preferably by at least 50 % by weight, preferably by at least 60 % by weight, further preferably by at least 70 % by weight, preferably by at least 80 % by weight, particularly preferably by at least 90 % by weight, of acrylamide, based on the total weight of the aqueous preparation,
and/or
wherein the aqueous preparation with reduced acrylamide content obtained in step (iv) has an acrylamide content of less than 2000 µg/kg, preferably of less than 850 mg/kg and particularly preferably of less than 500 µg/kg based on the dry mass of the preparation
and/or
wherein the aqueous preparation obtained in step (iv) is further processed in step (v) and wherein the further processing comprises at least one process step selected from the group consisting of drying, evaporating, concentrating, freeze-drying, fluidized bed drying, spray-drying, granulating, comminuting and filtering.

## Revendications

1. Procédé d'élimination ou de réaction d'acrylamide d'une préparation aqueuse pour produire un produit final à teneur réduite en acrylamide, comprenant les étapes suivantes consistant à:
(i) fournir une préparation aqueuse contenant de l'acrylamide et d'autres composants comme produit de départ pour produire un produit final à teneur réduite en acrylamide ;
(ii) diviser la préparation aqueuse en deux flux de matière pour obtenir un rétentat (R1) et un perméat (P1), le rétentat (R1) contenant les autres composants (K1), et le perméat (P1) contenant de l'acrylamide et de préférence aucun autre composant ou une faible proportion d'autres composants (K2) ;
(iii) mettre en contact le perméat (P1) avec un biocatalyseur réducteur d'acrylamide et obtenir un perméat (P2) à teneur réduite en acrylamide ;
(iv) combiner le perméat obtenu (P2) à teneur réduite en acrylamide et le rétentat (R1) de l'étape (ii) et obtenir une préparation aqueuse à teneur réduite en acrylamide, dans lequel le perméat (P2) ne contient pas de biocatalyseur;
(v) en option, traiter davantage la préparation aqueuse à teneur réduite en acrylamide de l'étape (iv) afin d'obtenir un produit final.

2. Procédé selon la revendication 1, dans lequel la séparation des flux de matière à l'étape (ii) est réalisée en tant que filtration, de préférence en tant que filtration membranaire.

3. Procédé selon l'une quelconque des revendications 1 ou 2, dans lequel la séparation des flux de matière à l'étape (ii) est réalisée en tant que filtration membranaire et la taille de pores de la membrane est d₉₀ ≤ 100 kDa, de préférence d₉₀ ≤ 50 kDa, de manière particulièrement préférée d₉₀ ≤ 30 kDa
et/ou
dans lequel la pression transmembranaire se situe et/ou est maintenue entre 0,01 mbar et 10 bar, de préférence entre 0,1 mbar et 7,5 bar, de préférence encore entre 1 mbar et 5 bar, de manière particulièrement préférée entre 500 mbar et 2,5 bar.

4. Procédé selon l'une quelconque des revendications précédentes, dans lequel le produit final est un produit final servant au plaisir, à la nutrition ou cosmétique, et est de préférence choisi dans le groupe comprenant les produits de pommes de terre sautés ou frits, les produits à base de maïs, les produits à base de café, les substituts de café, les snacks, les produits à base de blé, les cosmétiques, les pâtisseries telles que les biscuits, les biscottes, les barres de céréales, les scones, les cornets de crème glacée, les gaufres, les crumpets, le pain d'épices, le pain croustillant et les substituts de pain, les pâtes, le riz, les produits à base de poisson, les produits à base de viande, les céréales, la bière ou les aliments de complément pour enfants et nourrissons.

5. Procédé selon l'une quelconque des revendications précédentes, dans lequel le biocatalyseur est présent en tant que micro-organisme vivant et/ou en tant que micro-organisme inactivé et/ou comme lysat cellulaire et/ou comme enzyme (partiellement) purifiée et/ou comme enzyme immobilisée.

6. Procédé selon l'une quelconque des revendications précédentes, dans lequel le biocatalyseur est présent comme enzyme (partiellement) purifiée et/ou comme enzyme immobilisée qui est une amidase.

7. Procédé selon l'une quelconque des revendications précédentes, dans lequel le biocatalyseur est présent comme enzyme (partiellement) purifiée et/ou comme enzyme immobilisée qui comprend ou se compose (d')une séquence d'acides aminés avec une identité de séquence d'au moins 60 %, 70 %, 80 %, 90 %, 91 %, 92 %, 93 %, 94 %, 95 %, 96 %, 97 %, 98 % ou 99 %, de préférence d'au moins 90 %, 91 %, 92 %, 93 %, 94 %, 95 %, 96 %, 97 %, 98 % ou 99 %, par rapport à la longueur totale d'une enzyme avec une séquence choisie dans le groupe constitué des séquences selon SEQ ID NO. 1 à SEQ ID NO. 44.

8. Procédé selon l'une quelconque des revendications précédentes, dans lequel, à l'étape (iii), la mise en contact du perméat (P1) avec le biocatalyseur se fait par mélange, et puis ce mélange est divisé en deux flux de matière pour obtenir le perméat (P2), dans lequel le perméat (P2) ne contient pas de biocatalyseur.

9. Procédé selon l'une quelconque des revendications précédentes, dans lequel la mise en contact du perméat (P1) avec le biocatalyseur se fait via la surface de contact d'une membrane.

10. Procédé selon l'une quelconque des revendications précédentes, dans lequel l'étape (iii) comprend les sous-étapes suivantes consistant à:
(iii-1) mettre en contact le perméat (P1) avec un biocatalyseur réducteur d'acrylamide et obtenir un perméat (P2) ayant une teneur réduite en acrylamide, dans lequel le perméat (P2) comprend le biocatalyseur;
(iii-2) séparer le biocatalyseur du perméat (P2) de l'étape (iii-1) au moyen d'une séparation via une membrane (M2) et obtenir un rétentat (R2) qui comprend le biocatalyseur et obtenir le perméat (P2) ayant une teneur réduite en acrylamide, dans lequel le perméat (P2) ne comprend pas de biocatalyseur.

11. Procédé selon l'une quelconque des revendications précédentes, de préférence selon la revendication 10, dans lequel la division de la préparation aqueuse en deux flux de matière à l'étape (ii) et la séparation du biocatalyseur avant l'étape (iv) se font via une seule membrane (M1).

12. Procédé selon la revendication 10 ou 11, dans lequel les étapes (ii) et (iii-2) du procédé selon l'invention sont réalisées via une seule membrane (M1).

13. Procédé selon l'une quelconque des revendications 10 à 12, dans lequel les tailles de pores de la membrane (M1) sont inférieures ou égales à la taille de pores de la membrane (M2), et les tailles de pores de la membrane (M1) et de la membrane (M2) sont chacune inférieures au biocatalyseur.

14. Procédé selon l'une quelconque des revendications 10 à 13, dans lequel la taille de pores de la membrane, de préférence de la membrane (M1), est d₉₀ ≤ 100 kDa, de préférence d₉₀ ≤ 50 kDa, de manière particulièrement préférée d₉₀ ≤ 30 kDa, de manière tout particulièrement préférée d₉₀ ≤ 10 kDa
et/ou
dans lequel la pression transmembranaire se situe et/ou est maintenue entre 0,01 mbar et 10 bar, de préférence entre 0,1 mbar et 7,5 bar, de préférence encore entre 1 mbar et 5 bar, de manière particulièrement préférée entre 500 mbar et 2,5 bar.

15. Procédé selon l'une quelconque des revendications précédentes, dans lequel la préparation aqueuse à teneur réduite en acrylamide qui est obtenue à l'étape (iv) présente, par rapport à la préparation aqueuse fournie à l'étape (i), une teneur en acrylamide réduite d'au moins 20 % en poids, de préférence d'au moins 30 % en poids, de préférence encore d'au moins 40 % en poids, de préférence encore d'au moins 50 % en poids, de préférence d'au moins 60 % en poids, de préférence encore d'au moins 70 % en poids, de préférence d'au moins 80 % en poids, et de préférence encore d'au moins 90 %, par rapport au poids total de la préparation aqueuse,
et/ou
dans lequel la préparation aqueuse à teneur réduite en acrylamide qui est obtenue à l'étape (iv) présente une teneur en acrylamide inférieure à 2000 µg/kg, de préférence inférieure à 850 mg/kg et de manière particulièrement préférée inférieure à 500 µg/kg, par rapport à la masse sèche de la préparation
et/ou
dans lequel la préparation aqueuse obtenue à l'étape (iv) est traitée ultérieurement à l'étape (v), et dans lequel le traitement ultérieur comprend au moins une étape de procédé choisie dans le groupe constitué par le séchage, l'évaporation, la concentration, la lyophilisation, le séchage en lit fluidisé, le séchage par pulvérisation, la granulation, la fragmentation et la filtration.
